(19) Europäisches Patentamt
European Patent Office
Office européen des brevets

(11) EP 3 112 373 B1

(12) EUROPÄISCHE PATENTSCHRIFT

(45) Veröffentlichungstag und Bekanntmachung des Hinweises auf die Patenterteilung:
**30.08.2017 Patentblatt 2017/35**

(21) Anmeldenummer: **16180794.6**

(22) Anmeldetag: **10.04.2014**

(51) Int Cl.:
*C07F 9/6574* (2006.01)
*C07C 67/347* (2006.01)
*C07B 41/06* (2006.01)
*C07C 45/50* (2006.01)
*C07F 9/6584* (2006.01)
*C07F 15/00* (2006.01)
*C07F 9/145* (2006.01)
*C07F 9/146* (2006.01)
*C07F 9/24* (2006.01)
*B01J 31/24* (2006.01)
*B01J 31/18* (2006.01)
*B01J 31/20* (2006.01)
*B01J 31/22* (2006.01)

(54) **NEUER VIERZÄHNIGER PHOSPHOR-LIGAND MIT HOSTANOX O3 LEITSTRUKTUR**

NOVEL TETRADENTATE PHOSPHORUS LIGAND WITH HOSTANOX O3 GUIDE STRUCTURE

NOUVEAU LIGAND TETRADENTATE DE PHOSPHORE AYANT UNE STRUCTURE O3 D'HOSTANOX

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **03.05.2013 DE 102013208097**
**04.04.2014 DE 102014206520**

(43) Veröffentlichungstag der Anmeldung:
**04.01.2017 Patentblatt 2017/01**

(62) Dokumentnummer(n) der früheren Anmeldung(en) nach Art. 76 EPÜ:
**14717438.7 / 2 991 996**

(73) Patentinhaber: **Evonik Degussa GmbH**
**45128 Essen (DE)**

(72) Erfinder:
- **Dyballa, Katrin Marie**
  **45657 Recklinghausen (DE)**
- **Franke, Robert**
  **45772 Marl (DE)**
- **Fridag, Dirk**
  **45721 Haltern am See (DE)**
- **Hess, Dieter**
  **45770 Marl (DE)**
- **Hamers, Bart**
  **5961 RM Horst (NL)**
- **Börner, Armin**
  **18059 Rostock (DE)**
- **Selent, Detlef**
  **18059 Rostock (DE)**

(56) Entgegenhaltungen:
- **CHRISTOPHE M. THOMAS ET AL: "New Diphosphine Ligands Containing Ethyleneglycol and Amino Alcohol Spacers for the Rhodium-Catalyzed Carbonylation of Methanol", CHEMISTRY - A EUROPEAN JOURNAL, Bd. 8, Nr. 15, 2. August 2002 (2002-08-02) , Seite 3343, XP055117362, ISSN: 0947-6539, DOI: 10.1002/1521-3765(20020802)8:15<3343::AID-CHEM3343>3.0.CO;2-Z**

EP 3 112 373 B1

Anmerkung: Innerhalb von neun Monaten nach Bekanntmachung des Hinweises auf die Erteilung des europäischen Patents im Europäischen Patentblatt kann jedermann nach Maßgabe der Ausführungsordnung beim Europäischen Patentamt gegen dieses Patent Einspruch einlegen. Der Einspruch gilt erst als eingelegt, wenn die Einspruchsgebühr entrichtet worden ist. (Art. 99(1) Europäisches Patentübereinkommen).

## Beschreibung

Die Erfindung betrifft einen neuen vierzähnigen Phosphor-Liganden mit einer Hostanox 03 (Bis[3,3-bis-(4'-hydroxy-3'-tert-butylphenyl)butanosäure]-glycol ester bzw. Ethylen-bis[3,3-bis(3-tert-butyl-4-hydroxyphenyl)butyrat]) Leitstruktur. Offenbart ist auch ein Verfahren zu seiner Herstellung und Verwendung in der Hydroformylierung.

Die Reaktionen zwischen Olefinverbindungen, Kohlenmonoxid und Wasserstoff in Gegenwart eines Katalysators zu den um ein C-Atom reicheren Aldehyden ist als Hydroformylierung bzw. Oxierung bekannt. Als Katalysatoren in diesen Reaktionen werden häufig Verbindungen der Übergangsmetalle der VIII. Gruppe des Periodensystems der Elemente verwendet. Bekannte Liganden sind beispielsweise Verbindungen aus den Klassen der Phosphine, Phosphite und Phosphonite mit jeweils dreiwertigem Phosphor $P^{III}$. Eine Übersicht über den Stand der Hydroformylierung von Olefinen findet sich beispielsweise in B. CORNILS, W. A. HERRMANN, "Applied Homogeneous Catalysis with Organometallic Compounds", Vol. 1 & 2, VCH, Weinheim, New York, 1996 bzw. R. Franke, D. Selent, A. Börner, "Applied Hydroformylation", Chem. Rev., 2012, DOI:10.1021/cr3001803.

Jede katalytisch aktive Zusammensetzung hat ihre spezifischen Vorzüge. Je nach Einsatzstoff und Zielprodukt kommen daher unterschiedliche katalytisch aktive Zusammensetzungen zum Einsatz. Die Patente US 4 694 109 und US 4 879 416 beschreiben Bisphosphinliganden und ihren Einsatz in der Hydroformylierung von Olefinen bei niedrigen Synthesegasdrücken. Besonders bei der Hydroformylierung von Propen werden mit Liganden dieses Typs hohe Aktivitäten und hohe n/i-Selektivitäten (n/i = das Verhältnis von linearem Aldehyd (= n) zu verzeigtem (= iso) Aldehyd)) erreicht. In WO 95/30680 werden zweizähnige Phosphinliganden und ihr Einsatz in der Katalyse, unter anderem auch in Hydroformylierungsreaktionen, beschrieben.

In der DE 101 40 083 A1 werden Phosphitliganden. genauer Bisphosphitliganden, beschrieben, das Phosphoratom ist also direkt an drei Sauerstoffatome gebunden. Mindestens einer der drei Sauerstoffatome ist hierbei das Sauerstoffatom einer Estergruppe. Das Phosphoratom und die COO-Gruppe bilden hierbei einen Heterocyclus aus. Die Phosphoratome der auf Seite 7 dargestellten Verbindungen (H) und (L) sind über eine Gruppe verknüpft, welche zwei Estergruppen aufweist, wobei das innere der Brücke den Säurerest des Diesters darstellt. Das äußere Ende der Brücke stellt jeweils eine COO-Gruppe dar, welche direkt an das Phosphoratom anknüpft.

CHEMISTRY - A EUROPEAN JOURNAL, Bd. 8, Nr. 15, 2002, Seite 3343 offenbart zweizähnige Phosphinliganden und ihre Verwendung in der Hydroformylierung.

Eigene Versuche haben ergeben, dass diese Art der Liganden aufgrund der dort vorliegenden Esterfuktionalität besonders hydrolyseempfindlich ist, und sich die Liganden durch eine geringe Stabilität in einem großtechnischen Prozess auszeichnen. Daher sollten derartige Carbonsäurestrukturen in Phosphitliganden vermieden werden.

Der Nachteil von zwei- und mehrzähnigen Phosphinliganden ist ein relativ hoher Aufwand, der zu ihrer Darstellung notwendig ist. Daher ist es oftmals nicht rentabel, solche Systeme in technischen Prozessen einzusetzen. Hinzu kommt eine vergleichsweise geringe Aktivität, die durch hohe Verweilzeiten reaktionstechnisch kompensiert werden müssen. Dies wiederum führt zu unerwünschten Nebenreaktionen der Produkte.

Katalytisch aktive Zusammensetzungen auf Basis von Rhodium-Bisphosphit-Komplexen sind geeignet für die Hydroformylierung von linearen Olefinen mit end- und innenständigen Doppelbindungen. Diese Phosphite ergeben bei ihrer Koordination an ein Übergangsmetallzentrum Katalysatoren mit einer gesteigerten Aktivität, was gleichbedeutend mit einer höheren Ausbeute ist. Jedoch ist das Standzeitverhalten dieser katalytisch aktiven Zusammensetzungen, unter anderem wegen der Hydrolyseempfindlichkeit der Phosphitliganden, unbefriedigend.

Neben dem Bestreben einer hohen Reaktivität und n/i-Selektivität in Bezug auf die zu carbonylierenden, ungesättigten Verbindungen ist die Stabilität - konkret die Standzeit - der katalytisch aktiven Zusammensetzung aus jeweils verwendetem Metall, Liganden sowie weiteren Komponenten mit aktivierender Wirkung mit Blick auf die als Liganden eingesetzten Bisphophite eine ständige Aufgabe der Forschung. Dies gilt insbesondere hinsichtlich olefinhaltiger Gemische, speziell in der Hydroformylierung von Gemischen linearer Olefine.

In der großtechnischen Hydroformylierung werden heute phosphororganische Metall-Komplexkatalysatoren auf Cobalt- oder Rhodiumbasis verwendet. Die Katalysatoren werden homogen in der flüssigen Hydroformylierungsmischung gelöst. Im Rahmen der Ausscheidung des Zielprodukts (der Aldehyde) aus dem Hydroformylierungsgemisch ist auch der Homogenkatalysator aus dem Hydroformylierungsgemisch schonend abzutrennen, da der Komplexkatalysator vergleichsweise empfindlich auf Zustandsänderungen reagiert und seine Aktivität verlieren könnte.

Insbesondere die Abtrennung von Rh-basierten Katalysatorkomplexen aus homogen katalysierten Hydroformylierungsmischungen erweist sich als technisch anspruchsvoll. Dies liegt unter anderem daran, dass Rh ein sehr teures Edelmetall ist, dessen Verlust es tunlichst zu vermeiden gilt. Aus diesem Grunde muss das Rhodium aus dem Produktstrom möglichst vollständig abgetrennt und zurückgewonnen werden. Da die Rh-Konzentration in typischen Hydroformylierungsreaktionen lediglich 20 bis 100 ppm beträgt und eine typische "world scale" Oxo-Anlage eine Jahresleistung von 200 000 Tonnen erzielt, müssen Trennapparate eingesetzt werden, die einerseits einen großen Durchsatz erlauben und andererseits das nur in geringen Mengen enthaltene Rh sicher abscheiden. Erschwerend kommt hinzu, dass die zum Katalysator-Komplex gehörigen Organo-PhosphorLiganden sehr empfindlich auf Zustandsänderungen

reagieren und rasch desaktivieren. Ein desaktivierter Katalysator ist bestenfalls nur aufwändig zu reaktivieren. Die Katalysatorabscheidung muss deshalb besonders schonend erfolgen.

Traditionell wird der Katalysator destillativ aus dem Hydroformylierungsgemisch abgeschieden. Um die Gefahr der Desaktivierung zu senken und den Energieverbrauch des Prozesses zu senken gibt es in jüngerer Zeit Bestrebungen, den homogen gelösten Katalysator mit Hilfe von Membrantechnologie (Nanofiltration) aus dem Hydroformylierungsgemisch abzutrennen.

Die Grundlagen der membrangestützten, organophilen Nanofiltration zur Abscheidung von homogen gelösten Katalysatorkomplexen aus Hydroformylierungsmischungen beschreiben Priske, M. et al.: "Reaction integrated separation of homogeneous catalysts in the hydroformylation of higher olefins by means of organophilic nanofiltration". Journal of Membrane Science, Volume 360, Issues 1-2, 15 September 2010, Pages 77-83; doi:10.1016/j.memsci.2010.05.002. Priske et al. beschreiben die Abtrennung von freien Liganden und aktivem Katalysator.

Die bisher im Stand der Technik verwendeten Liganden zeichnen sich vor allem in der freien Form, d.h. wenn sie nicht an ein Metallatom komplexiert sind, durch einen nur geringen Rückhalt aus.

Da Liganden in der Regel in einem Überschuss zum Metall in der Reaktionsmischung vorliegen, ist es somit wünschenswert, auch den freien Liganden nach der Reaktion aus dem Produktgemisch abzutrennen.

Ein weiterer Nachteil der bisher im Stand der Technik beschriebenen Liganden liegt darin, dass in der Regel ein großer Ligandenüberschuss in der Reaktionsmischung zugegen sein muss.

Die technische Aufgabe, welche der vorliegenden Erfindung zugrunde lag, ist die Bereitstellung eines neuen Liganden, welcher in der Hydroformylierung von ungesättigten Verbindungen nicht die aus dem Stand der Technik zuvor aufgezeigten Nachteile aufweist.

Die Eigenschaften des Liganden sollen mindestens in einem der folgenden Punkte verbessert werden:

1) Aktivität / Ausbeute
2) n-Regioselektivität in Bezug auf die Hydroformylierung,
3) Abtrennbarkeit.

Die Aufgabe wird gelöst durch einen Liganden nach Anspruch 1. Hierbei handelt es sich um einen Tetraphosphitliganden. Offenbart ist auch ein Ligand, welcher eine der allgemeinen Strukturen **A, B** oder **C** aufweist:

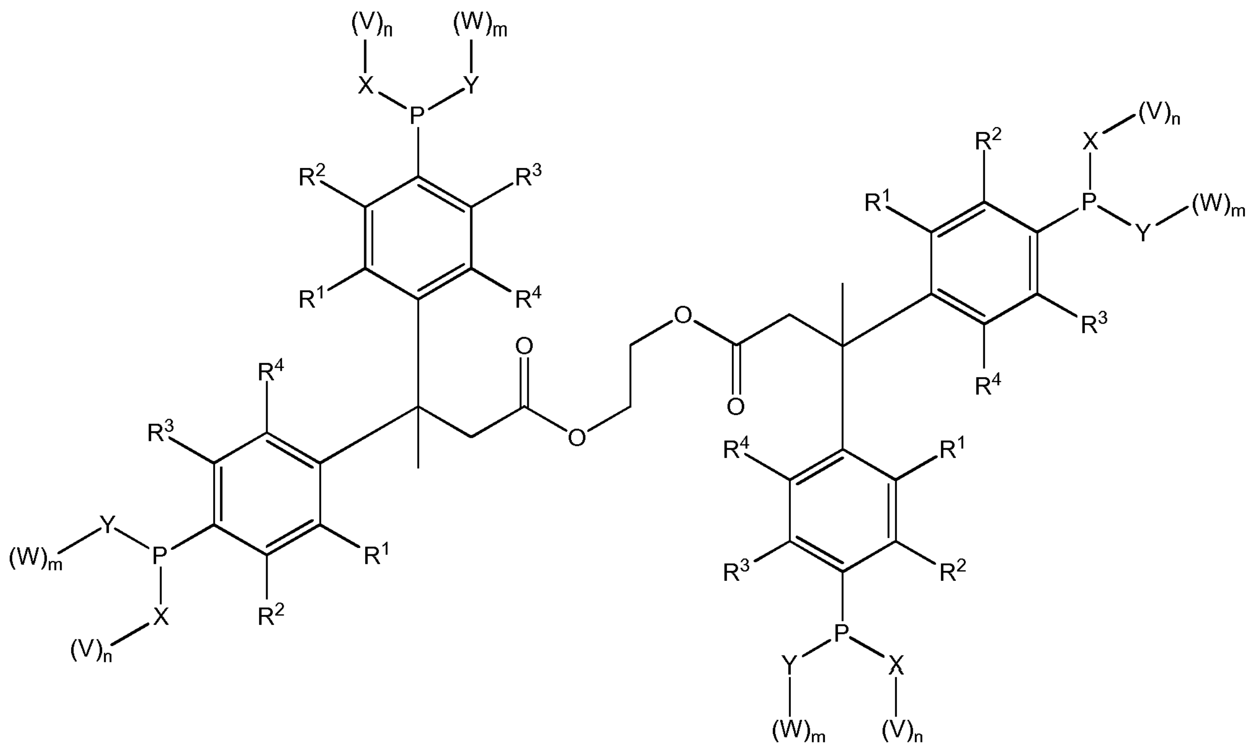

**A**

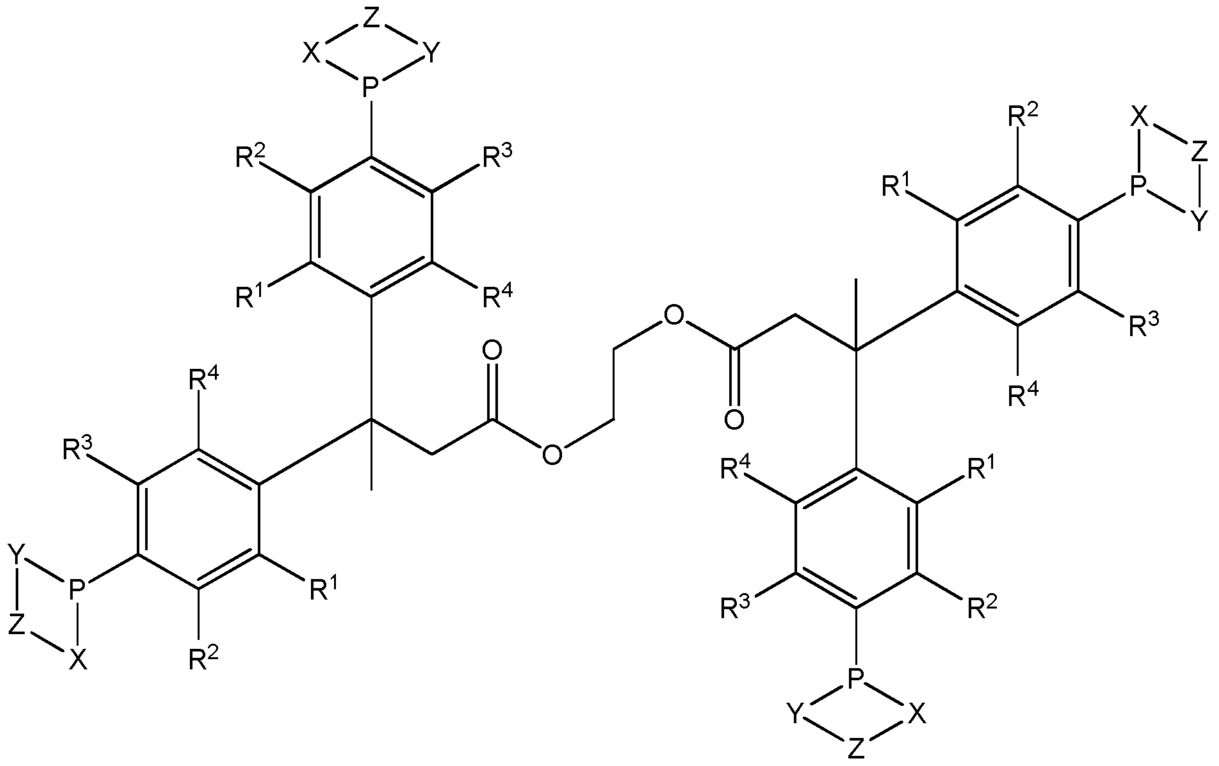

**B**

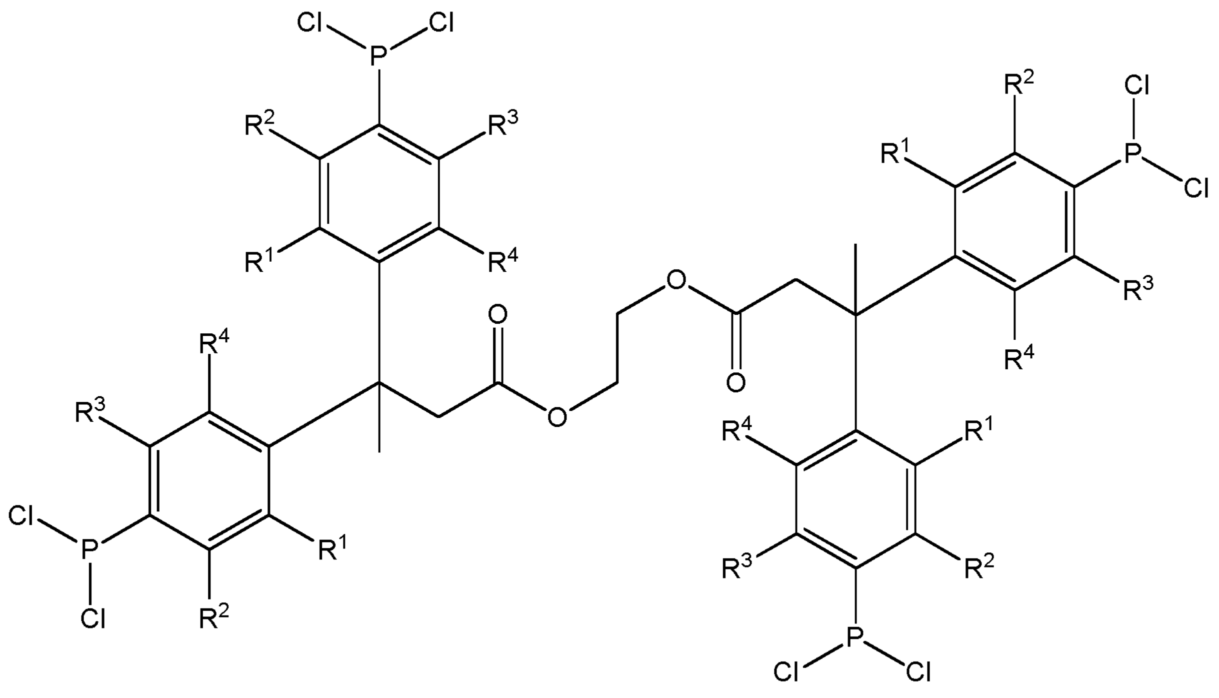

**C**

wobei

$R^1$, $R^2$, $R^3$, $R^4$ jeweils unabhängig voneinander ausgewählt sind aus: -H, -($C_1$-$C_{12}$)-Alkyl,
X und Y jeweils unabhängig voneinander für O oder N stehen können,
für den Fall, dass X für O steht n = 1 ist,
für den Fall, dass Y für O steht m = 1 ist,

für den Fall, dass X für N steht n = 1 oder 2 ist,
für den Fall, dass Y für N steht m = 1 oder 2 ist,
V und W jeweils unabhängig voneinander ausgewählt sind aus:

-H, -($C_1$-$C_{12}$)-Alkyl, -($C_1$-$C_{12}$)-Heteroalkyl, -($C_4$-$C_{20}$)-Aryl, -($C_4$-$C_{20}$)-Aryl-($C_1$-$C_{12}$)-Alkyl, -($C_4$-$C_{20}$)-Aryl-O-($C_1$-$C_{12}$)-Alkyl, -($C_1$-$C_{12}$)-Alkyl-($C_4$-$C_{20}$)-Aryl, -($C_4$-$C_{20}$)-Aryl-COO-($C_1$-$C_{12}$)-Alkyl, -($C_4$-$C_{20}$)-Aryl-CONH-($C_1$-$C_{12}$)-Alkyl, , -($C_4$-$C_{20}$)-Aryl-CON[($C_1$-$C_{12}$)-Alkyl]$_2$, -($C_4$-$C_{20}$)-Heteroaryl, -($C_4$-$C_{20}$)-Heteroaryl-($C_1$-$C_{12}$)-Alkyl, -($C_3$-$C_{12}$)-Cycloalkyl, -($C_3$-$C_{12}$)-Cycloalkyl-($C_1$-$C_{12}$)-Alkyl, -($C_3$-$C_{12}$)-Heterocycloalkyl, -($C_3$-$C_{12}$)-Heterocycloalkyl-($C_1$-$C_{12}$)-Alkyl, -CO-($C_1$-$C_{12}$)-Alkyl, -CO-($C_4$-$C_{20}$)-Aryl, -CO-($C_4$-$C_{20}$)-Heteroalkyl, -($C_4$-$C_{20}$)-Aryl-CO-($C_1$-$C_{12}$)-Alkyl, -($C_4$-$C_{20}$)-Aryl-CO-($C_4$-$C_{20}$)-Aryl,

Z ausgewählt ist aus:

-($C_1$-$C_{14}$)-Alkyl-, -($C_4$-$C_{20}$)-Aryl-, -($C_4$-$C_{20}$)-Aryl-($C_1$-$C_{12}$)-Alkyl-, -($C_1$-$C_{14}$)-Heteroalkyl-, -($C_4$-$C_{20}$)-Heteroaryl-, -CO-($C_4$-$C_{14}$)-Heteroaryl-, -CO-($C_4$-$C_{20}$)-Aryl-, -($C_3$-$C_{12}$)-Cycloalkyl-, -($C_3$-$C_{12}$)-Heterocycloalkyl-, -($C_4$-$C_{20}$)-Aryl-CO-($C_4$-$C_{20}$)-Aryl-, -($C_1$-$C_{14}$)-Alkyl-O-($C_1$-$C_{14}$)-Alkyl-, wobei die genannten Alkyl-, Heteroalkyl-, Cycloalkyl-, Heterocycloalkyl-, Aryl- und Heteroarylgruppen optional ein oder mehrfach substituiert sind.

Alkyl steht für einen nicht verzweigten oder verzweigten aliphatischen Rest.
Aryl für aromatische (Kohlenwasserstoff-)Reste, vorzugsweise mit bis zu 14 C-Atomen, z. B. Phenyl- ($C_6H_5$-), Naphthyl- ($C_{10}H_7$-), Anthryl- ($C_{14}H_9$-), Binaphthyl vorzugsweise Phenyl. Cycloalkyl für gesättigte cyclische Kohlenwasserstoffe, die ausschließlich Kohlenstoff-Atome im Ring enthalten.
Heteroalkyl für einen nicht verzweigten oder verzweigten aliphatischen Rest, der ein bis vier, bevorzugt ein oder zwei, Heteroatome ausgewählt aus der Gruppe bestehend aus N, O, S und substituiertem N enthalten kann.
Heteroaryl für einen Arylrest, in dem ein bis vier, bevorzugt ein oder zwei, Kohlenstoffatome durch Heteroatome ausgewählt aus der Gruppe bestehend aus N, O, S und substituiertem N ersetzt sein können, wobei der Heteroarylrest auch Teil einer größeren kondensierten Ringstruktur sein kann.
Heterocycloalkyl für gesättigte cyclische Kohlenwasserstoffe, die ein bis vier, bevorzugt ein oder zwei, Heteroatome ausgewählt aus der Gruppe bestehend aus N, O, S und substituiertem N enthalten kann.

Unter Heteroarylrest, der Teil einer kondensierten Ringstruktur sein kann, werden bevorzugt Systeme verstanden, in denen kondensierte Fünf- oder Sechsringe gebildet werden, z.B. Benzofuran, Isobenzofuran, Indol, Isoindol, Benzothiophen, Benzo(c)thiophen, Benzimidazol, Purin, Indazol, Benzoxazol, Chinolin, Isochinolin, Chinoxalin, Chinazolin, Cinnolin, Acridin.

Die genannten substituierten N können einfach substituiert sein, die Alkyl-, Heteroalkyl-, Cycloalkyl-, Heterocycloalkyl-, Aryl- und Heteroarylgruppen können ein- oder mehrfach, besonders bevorzugt ein-, zwei- oder dreifach, substituiert sein.

Vorzugsweise sind die Substituenten ausgewählt aus: -H, -($C_1$-$C_{14}$)-Alkyl, -($C_1$-$C_{14}$)-Heteroalkyl, -($C_4$-$C_{20}$)-Aryl, -($C_4$-$C_{20}$)-Heteroaryl, ($C_3$-$C_{12}$)-Cycloalkyl, -($C_3$-$C_{12}$)-Heterocycloalkyl, -$CF_3$, - Halogen (Fluor, Chlor, Brom, Iod), -OH, -($C_1$-$C_{12}$)-Alkoxy, -($C_4$-$C_{14}$)-Aryloxy, -($C_3$-$C_{14}$)-Heteroaryloxy, -O-($C_1$-$C_{14}$)-Alkyl, -O-($C_4$-$C_{20}$)-Aryl, -Sulfonyl, -N[($C_1$-$C_{14}$)-Alkyl]$_2$, -N[($C_4$-$C_{14}$)-Aryl]$_2$, -N[($C_1$-$C_{14}$)-Alkyl][($C_4$-$C_{14}$)-Aryl], -COO-($C_1$-$C_{14}$)-Alkyl, -COO-($C_4$-$C_{20}$)-Aryl, -COO-N[($C_1$-$C_{12}$)-Alkyl)]$_2$.

Besonders bevorzugt sind die Substituenten ausgewählt aus: -H, -($C_1$-$C_{14}$)-Alkyl, -($C_1$-$C_{14}$)-Heteroalkyl, -($C_4$-$C_{20}$)-Aryl, -($C_4$-$C_{20}$)-Heteroaryl, ($C_3$-$C_{12}$)-Cycloalkyl, -($C_3$-$C_{12}$)-Heterocycloalkyl, -OH, -O-($C_1$-$C_{14}$)-Alkyl, -O-($C_4$-$C_{20}$)-Aryl, -COO-($C_1$-$C_{14}$)-Alkyl, -COO-($C_4$-$C_{20}$)-Aryl, -COO-N[($C_1$-$C_{12}$)-Alkyl)]$_2$.

Ganz besonders bevorzugt sind die Substituenten ausgewählt aus: -H, -($C_1$-$C_{14}$)-Alkyl, -($C_1$-$C_{14}$)-Heteroalkyl, -($C_4$-$C_{20}$)-Aryl, -OH, -O-($C_1$-$C_{14}$)-Alkyl, -O-($C_4$-$C_{20}$)-Aryl, -N[($C_1$-$C_{14}$)-Alkyl]$_2$, - COO-($C_1$-$C_{14}$)-Alkyl, -COO-($C_4$-$C_{20}$)-Aryl, -COO-N[($C_1$-$C_{12}$)-Alkyl)]$_2$.

In einer Ausführungsform sind V und W jeweils unabhängig voneinander ausgewählt aus: -H, -($C_1$-$C_{12}$)-Alkyl, -($C_1$-$C_{12}$)-Heteroalkyl, -($C_4$-$C_{20}$)-Aryl, -($C_4$-$C_{20}$)-Aryl-($C_1$-$C_{12}$)-Alkyl, -($C_4$-$C_{20}$)-Aryl-O-($C_1$-$C_{12}$)-Alkyl, -($C_1$-$C_{12}$)-Alkyl-($C_4$-$C_{20}$)-Aryl, -($C_4$-$C_{20}$)-Aryl-COO-($C_1$-$C_{12}$)-Alkyl, -($C_4$-$C_{20}$)-Heteroaryl, -($C_3$-$C_{12}$)-Cycloalkyl, -($C_3$-$C_{12}$)-Heterocycloalkyl, -($C_3$-$C_{12}$)-Heterocycloalkyl-($C_1$-$C_{12}$)-Alkyl, -CO-($C_1$-$C_{12}$)-Alkyl, -CO-($C_4$-$C_{20}$)-Aryl, -CO-($C_4$-$C_{20}$)-Heteroalkyl, -($C_4$-$C_{20}$)-Aryl-CO-($C_1$-$C_{12}$)-Alkyl, -($C_4$-$C_{20}$)-Aryl-CO-($C_4$-$C_{20}$)-Aryl.

In einer Ausführungsform sind V und W jeweils unabhängig voneinander ausgewählt aus:

-H, -($C_1$-$C_{12}$)-Alkyl, -($C_4$-$C_{20}$)-Aryl, -($C_4$-$C_{20}$)-Aryl-($C_1$-$C_{12}$)-Alkyl, -($C_4$-$C_{20}$)-Aryl-O-($C_1$-$C_{12}$)-Alkyl, ($C_1$-$C_{12}$)-Alkyl-($C_4$-$C_{20}$)-Aryl, -($C_4$-$C_{20}$)-Aryl-COO-($C_1$-$C_{12}$)-Alkyl.

In einer Ausführungsform ist Z ausgewählt aus:

$-(C_1-C_{14})$-Alkyl-, $-(C_4-C_{20})$-Aryl-, $-(C_4-C_{20})$-Aryl-$(C_1-C_{12})$-Alkyl-, $-(C_4-C_{20})$-Heteroaryl-, -CO-$(C_4-C_{14})$-Heteroaryl-, -CO-$(C_4-C_{20})$-Aryl-.

In einer Ausführungsform stehen $R^1$, $R^3$, $R^4$ für -H.
In einer Ausführungsform steht $R^2$ für $-(C_1-C_{12})$-Alkyl.
Hierbei ist *tert*-Butyl bevorzugt.
In einer Ausführungsform stehen X und Y für O.
In einer Ausführungsform stehen X und Y für N.
In einer Ausführungsform stehen X und Y für O, wobei dieses mit einem Phenylrest verknüpft ist, welcher wiederum substituiert sein kann, und auch Bestandteil eines größeren kondensierten aromatischen Systems sein kann.
Der Ligand zeichnet sich durch ein sehr hohes Molekulargewicht aus. Er kann aufgrund seiner Größe am Ende der Reaktion mittels Membranfiltration sehr gut aus dem Reaktionsgemisch abgetrennt werden.
Ferner zeichnen sich der Ligand durch ein deutlich geringeres Ligand zu Metall-Verhältnis in einem Komplex aus. Das heißt es handelt sich um einen Liganden, der mit wenig Phosphor schon das Metall stabilisieren kann.
Neben den Liganden wird auch ein Komplex offenbart, welcher den Liganden umfasst.
Komplex umfassend:

- einen zuvor beschriebenen Liganden,
- ein Metallatom ausgewählt aus: Rh, Ru, Co, Ir.

Hierbei ist Rh bevorzugt.
Siehe hierzu R. Franke, D. Selent, A. Börner, "Applied Hydroformylation", Chem. Rev., 2012, DOI:10.1021/cr3001803; S. 5688 Schema 12 "General Method for the Preparation of a P-Modified Rh precatalyst" und darin zitierte Literaturstellen sowie P. W. N. M. van Leeuwen, in Rhodium Catalyzed Hydroformylation, P. W. N. M. van Leeuwen, C. Claver (Hrsg.), Kluwer, Dordrecht, 2000 unter anderem S. 48 ff, S.233 ff. und darin zitierte Literaturstellen sowie K.D. Wiese und D. Obst in Top. Organomet. Chem. 2006, 18, 1-13; Springer Verlag Berlin Heidelberg 2006 S. 6 ff sowie darin zitierte Literaturstellen.
Des Weiteren wird die Verwendung eines zuvor beschriebenen Liganden zur Katalyse einer Hydroformylierungsreaktion offenbart. Offenbart wird auch das Verfahren zur Hydroformylierung von Olefinen, bei welchem der Ligand zum Einsatz kommt.
Verfahren umfassend die Verfahrensschritte:

a) Vorlegen eines Olefins,
b) Zugabe eines zuvor beschriebenen Komplexes,
oder eines zuvor beschriebenen Liganden und einer Verbindung, welche ein Metallatom ausgewählt aus: Rh, Ru, Co, Ir aufweist,
c) Zuführen von $H_2$ und CO,
d) Erwärmen des Reaktionsgemisches, wobei das Olefin zu einem Aldehyd umgesetzt wird,

Hierbei können die Verfahrensschritte a) bis d) in beliebiger Reihenfolge durchgeführt werden.
Die Reaktion wird bei üblichen Bedingungen durchgeführt.
Bevorzugt sind eine Temperatur von 80 °C bis 160 °C und ein Druck von 1 bis 300 bar. Besonders bevorzugt sind eine Temperatur von 100 °C bis 160 °C und ein Druck von 15 bis 250 bar.
In einer Variante des Verfahrens umfasst dieses den zusätzlichen Verfahrensschritt:

b') Zugabe eines freien zuvor beschriebenen Liganden.

In einer Variante des Verfahrens umfasst dieses den zusätzlichen Verfahrensschritt:

e) Abtrennung des Komplexes vom Reaktionsgemisch.

In einer Variante des Verfahrens umfasst dieses den zusätzlichen Verfahrensschritt:

e') Abtrennung des freien Liganden vom Reaktionsgemisch.

In einer Variante des Verfahrens erfolgt die Abtrennung mittels Membranfiltration.
In einer Variante des Verfahrens liegt der Rückhalt R der Membran für den Liganden im Bereich von 90 % bis 100 %, was gemäß der Formel $R = 1 - c_{Pei}/c_{Rei}$ einem Wert von 0,9 bzw. 1 entspricht. Vorzugsweise liegt der Rückhalt R der

Membran für den Liganden im Bereich von 95 % bis 100 %, was gemäß der Formel R = 1 - $c_{Pei}/c_{Rei}$ einem Wert von 0,95 bzw. 1 entspricht.

In einer Variante des Verfahrens ist das Olefin ausgewählt aus: Alkene, Cycloalkene, Carbonsäureester, aromatische Olefine mit jeweils einer Kohlenstoffanzahl von 2 bis 20.

Im Folgenden werden Ausführungsformen der Offenbarung anhand von Ausführungsbeispielen näher erläutert.

Allgemeine Arbeitsvorschriften

Alle nachfolgenden Präparationen wurden mit Standard-Schlenk-Technik unter Schutzgas durchgeführt. Die Lösungsmittel wurden vor Gebrauch über geeigneten Trocknungsmitteln getrocknet (Purification of Laboratory Chemicals, W. L. F. Armarego (Autor), Christina Chai (Autor), Butterworth Heinemann (Elsevier), 6. Auflage, Oxford 2009).

Phosphortrichlorid (Aldrich) wurde vor dem Einsatz unter Argon destilliert. Alle präparativen Arbeiten erfolgten in ausgeheizten Gefäßen. Die Charakterisierung der Produkte erfolgte mittels NMR-Spektroskopie. Chemische Verschiebungen werden in ppm angegeben. Die Referenzierung der $^{31}P$-NMR-Signale erfolgte gemäß: $SR_{31P} = SR_{1H} * (BF_{31P} / BF_{1H}) = SR_{1H} * 0{,}4048$. (Robin K. Harris, Edwin D. Becker, Sonia M. Cabral de Menezes, Robin Goodfellow, and Pierre Granger, Pure Appl. Chem., 2001, 73, 1795-1818; Robin K. Harris, Edwin D. Becker, Sonia M. Cabral de Menezes, Pierre Granger, Roy E. Hoffman and Kurt W. Zilm, Pure Appl. Chem., 2008, 80, 59-84).

Die Aufnahme von Kernresonanzspektren erfolgte an Bruker Avance 300 bzw. Bruker Avance 400, die gaschromatografische Analyse an Agilent GC 7890A, die Elementaranalyse an Leco TruSpec CHNS und Varian ICP-OES 715, und die ESI-TOF Massenspektrometrie an Thermo Electron Finnigan MAT 95-XP und Agilent 6890 N/5973 Geräten.

Hostanox 03 (Bis[3,3-bis-(4'-hydroxy-3'-tert-butylphenyl)butanosäure]-glycol ester bzw. Ethylenbis[3,3-bis(3-tert-butyl-4-hydroxyphenyl)butyrat]) ist kommerziell erhältlich und wurde von Clariant bezogen.

**Ligand I:**

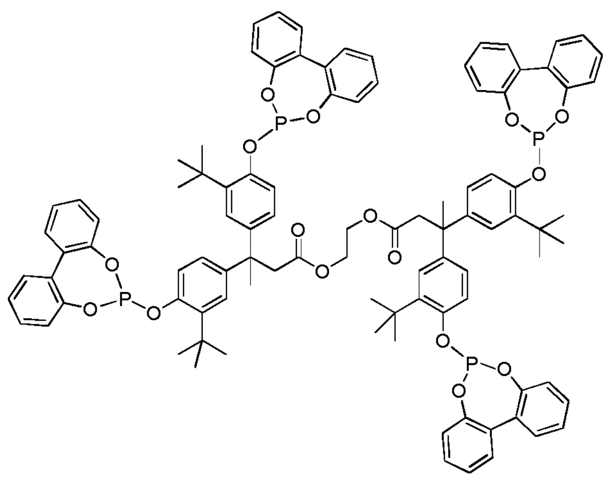

Eine gerührte Lösung von 6-Chlordibenzo[d,*f*][1,3,2]dioxaphosphepin (0,548 g; 2,188 mmol) in THF (4 ml) wird bei 0°C tropfenweise mit einer Mischung aus Hostanox 03 (Ethylenbis[3,3-bis(3-tert-butyl-4-hydroxyphenyl)butyrat]) (0,395 g; 0,497 mmol), Triethylamin (0,62 ml) und THF (6 ml) versetzt. Man lässt über Nacht bei Raumtemperatur rühren, filtriert, engt das Filtrat im Vakuum ein und trocknet den erhaltenen Rückstand 2 h bei 50 °C / 0,1 mbar. Die säulenchromatografische Reinigung (Hexan/Dichlormethan, 1:4, $R_f$ = 0,4) ergibt 0,449 g (0,272 mmol; 54%) des Tetraphosphites. Analyse (ber. für $C_{98}H_{94}O_{16}P_4$ = 1651,68 g/Mol): C 71,41 (71,26); H 5,73 (5,74); P 7,53 (7,50)%. $^{31}P$-NMR ($CD_2Cl_2$): 145,4 (s) ppm. $^{1}H$-NMR ($CD_2Cl_2$): 1,37 (36 H); 1,92 (6 H); 3,17 (4 H); 3,93 (4 H); 7,07...7,59 (44 H) ppm. ESI-TOF HRMS: m/e 1652,55888 $(M+H)^+$.

**Ligand II:**

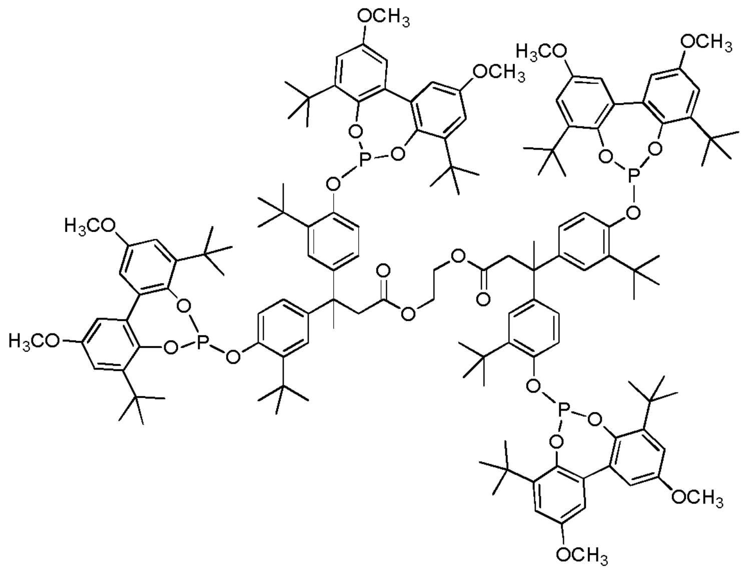

Eine gerührte Lösung von 4,8-Di-tert-butyl-6-chlor-2,10-dimethoxydibenzo[d,*f*][1,3,2]-dioxaphosphepin (2,387 g; 5,644 mmol) in THF (12 ml) wird bei 0 °C tropfenweise mit einer Mischung aus Hostanox 03 (Ethylenbis[3,3-bis(3-tert-butyl-4-hydroxyphenyl)butyrat]) (1,019 g; 1,282 mmol), Triethylamin (1,6 ml) und THF (12 ml) versetzt. Man lässt über Nacht bei Raumtemperatur rühren, filtriert, engt das Filtrat im Vakuum ein und trocknet den erhaltenen Rückstand 2 h bei 50 °C / 0,1 mbar. Die säulenchromatografische Reinigung (Hexan/Dichlormethan, 1:8, $R_f$ = 0,28) ergibt 1,437 g (0,614 mmol; 48%) des Tetraphosphites. Analyse (ber.für $C_{138}H_{174}O_{24}P_4$ = 2340,74 g/Mol): C 70,82 (70,81); H 7,57 (7,49); P 5,19 (5,29)%. $^{31}$P-NMR ($CD_2Cl_2$): 139,9 (s) ppm. $^{1}$H-NMR ($CD_2Cl_2$): 1,27 (36 H); 1,43 (72 H); 1,93 (6 H); 3,16 (4 H); 3,88 (24 H), 3,93 (4 H), 6,83...7,17 (28 H) ppm. ESI-TOF HRMS: *m/e* 2341,14642 $(M+H)^+$.

**Ligand III:** (entsprechend der Erfindung)

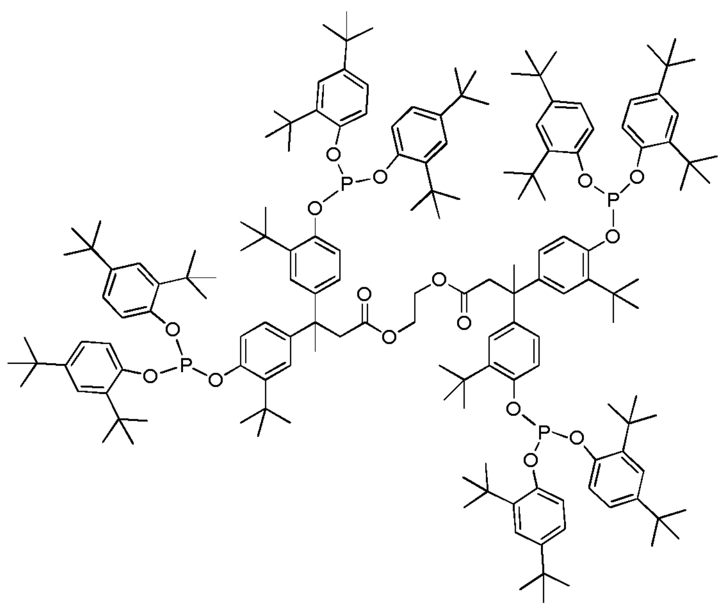

Eine gerührte Lösung von Bis(2,4-di-tert-butylphenyl)phosphorochloridit (3,381 g; 7,086 mmol) in THF (15 ml) wird bei 0 °C tropfenweise mit einer Mischung aus Hostanox 03 (Ethylenbis[3,3-bis(3-tert-butyl-4-hydroxyphenyl)butyrat]) (1,280 g; 1,61 mmol), Triethylamin (2 ml) und THF (15 ml) versetzt. Man lässt über Nacht bei Raumtemperatur rühren, filtriert, engt das Filtrat im Vakuum ein und trocknet den erhaltenen Rückstand 2 h bei 50 °C / 0,1 mbar. Die säulenchromatografische Reinigung (Hexan/Dichlormethan, 1:1, $R_f$ = 0,57) ergibt 3,6 g (1,41 mmol; 87%) des Tetraphosphites. Analyse (ber. für $C_{162}H_{230}O_{16}P_4$ = 2557,44 g/Mol): C 76,26 (76,08); H 9,05 (9,06); P 4,93 (4,84)%. $^{31}$P-NMR ($CD_2Cl_2$): 130,7 (s) ppm. $^1$H-NMR ($CD_2Cl_2$): 1,37 (72 H); 1,39 (36 H); 1,47 (72 H); 1,91 (6 H); 3,16 (4 H); 3,92 (4 H), 7,00...7,47 (36 H) ppm. ESI-TOF HRMS: *m/e* 518,37751; 647,46192; 1135,27566.

**Zwischenstufe IV:**

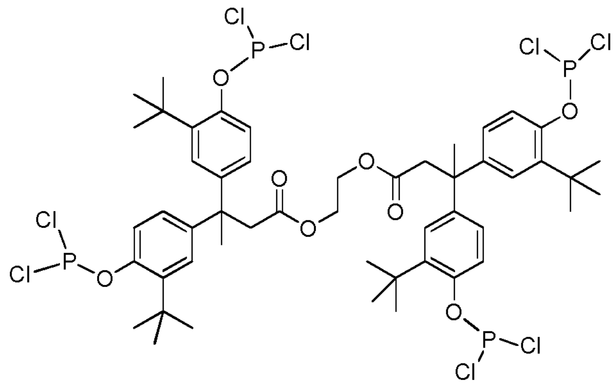

Eine Lösung von Phosphortrichlorid (0,66 g; 4,81 mmol) in THF (3 ml) wird bei 0 °C unter Rühren tropfenweise mit einer Mischung aus Hostanox 03 (Ethylenbis[3,3-bis(3-tert-butyl-4-hydroxyphenyl)butyrat]) (0,796 g; 1,001 mmol), Triethylamin (1,12 ml) und THF (30 ml) versetzt. Man lässt über Nacht bei Raumtemperatur rühren, filtriert, engt das Filtrat im Vakuum ein und trocknet den erhaltenen Rückstand 2 h bei 50 °C / 0,1 mbar. Das Produkt wird in Toluol (15 ml) gelöst, die Lösung über eine G4-Fritte filtriert und erneut zur Trockne eingeengt. Ausbeute: 0,935 g (0,78 mmol; 78%). Das so erhaltene Produkt wurde direkt weiterverwendet. $^{31}$P-NMR ($CD_2Cl_2$): 185,8 (s) ppm. $^1$H-NMR ($CD_2Cl_2$): 1,35 (36 H); 1,89 (6 H); 3,13 (4 H); 3,89 (4 H), 7,08...7,43 (12 H) ppm.

**Ligand V:**

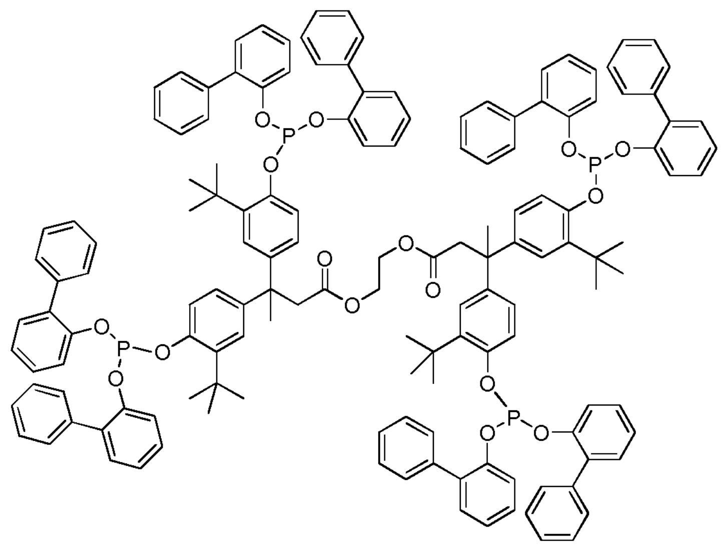

Eine Lösung der Zwischenstufe IV (0,441 g; 0,367 mmol) in Toluol (5 ml) wird bei 0 °C tropfenweise unter Rühren mit einer Lösung von 2-Phenylphenol (0,5 g; 2,938 mmol) in einer Mischung aus Toluol (7 ml) und Triethylamin (3,7 ml) versetzt. Man lässt über Nacht bei Raumtemperatur rühren, filtriert, engt das Filtrat im Vakuum ein und trocknet den erhaltenen Rückstand 2 h bei 50 °C / 0,1 mbar. Die säulenchromatografische Reinigung (Dichlormethan, $R_f$= 0,82)

ergibt 0,541 g (0,239 mmol; 65%) des Tetraphosphites. Analyse (ber. für $C_{146}H_{134}O_{16}P_4$ = 2268,54 g/Mol): C 77,34 (77,30); H 5,98 (5,96); P 5,85 (5,46)%. $^{31}P$-NMR ($CD_2Cl_2$): 129,9 (s) ppm. $^{1}H$-NMR ($CD_2Cl_2$): 1,16 (36 H); 1,85 (6 H); 3,06 (4 H); 3,89 (4 H), 6,76...7,44 (84 H) ppm. ESI-TOF HRMS: *m/e* 2290,8538 (M+Na)$^+$.

**Ligand VI:**

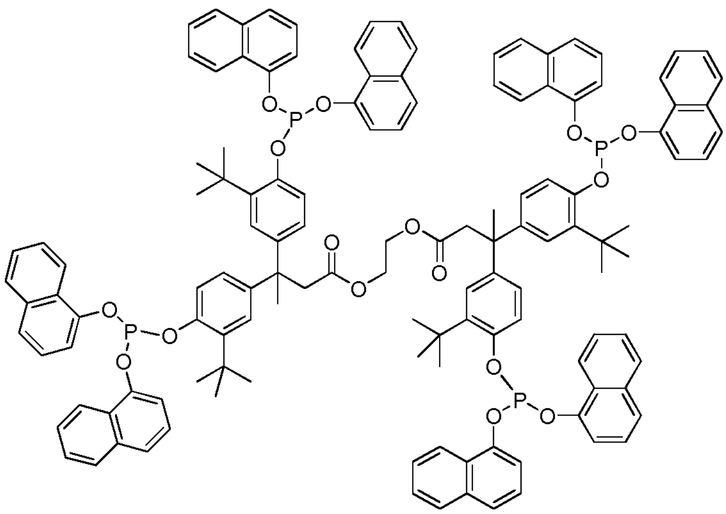

Eine Lösung der Zwischenstufe IV (0,612 g; 0,511 mmol) in Toluol (5 ml) wird bei 0 °C tropfenweise unter Rühren mit einer Lösung von 1-Naphthol (0,589 g; 4,084 mmol) in einer Mischung aus Toluol (12 ml) und Triethylamin (5,2 ml) versetzt. Man lässt über Nacht bei Raumtemperatur rühren, filtriert, engt das Filtrat im Vakuum ein und trocknet den erhaltenen Rückstand 2 h bei 50 °C / 0,1 mbar. Die säulenchromatografische Reinigung (Dichlormethan, $R_f$= 0,77) ergibt 0,629 g (0,305 mmol; 60%) des Tetraphosphites. Analyse (ber. für $C_{130}H_{118}O_{16}P_4$ = 2060,24 g/Mol): C 75,67 (75,79); H 5,99 (5,77); P 6,01 (6,01)%. $^{31}P$-NMR ($CD_2Cl_2$): 131,3 (s) ppm. $^{1}H$-NMR ($CD_2Cl_2$): 1,33 (36 H); 1,89 (6 H); 3,11 (4 H); 3,87 (4 H), 6,98...8,19 (84 H) ppm. ESI-TOF HRMS: *m/e* 2082,72565 (M+Na)$^+$.

**Ligand VII:**

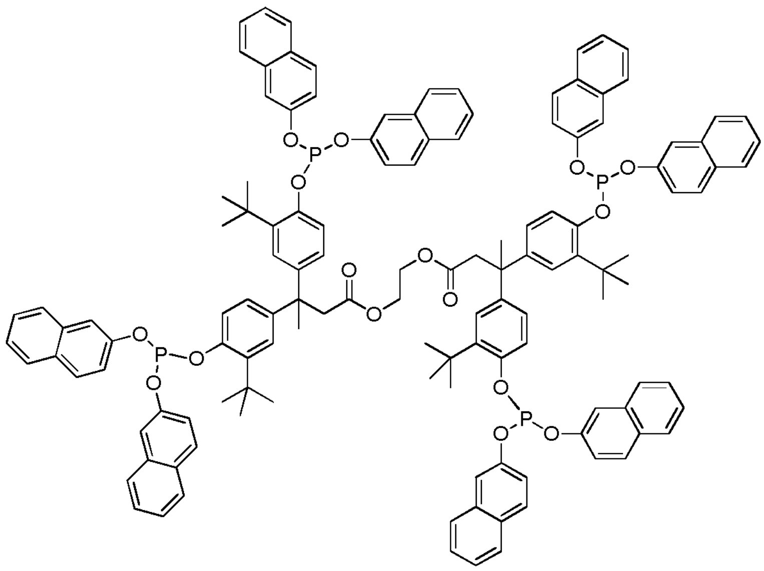

Eine Lösung der Zwischenstufe IV (0,612 g; 0,511 mmol) in Toluol (5 ml) wird bei 0 °C tropfenweise unter Rühren mit einer Lösung von 1-Naphthol (0,589 g; 4,084 mmol) in einer Mischung aus Toluol (12 ml) und Triethylamin (5,2 ml) versetzt. Man lässt über Nacht bei Raumtemperatur rühren, filtriert, engt das Filtrat im Vakuum ein und trocknet den erhaltenen Rückstand 2 h bei 50 °C / 0,1 mbar. Die säulenchromatografische Reinigung (Dichlormethan, $R_f$= 0,82) ergibt 0,57 g (0,277 mmol; 54%) des Tetraphosphites. Analyse (ber. für $C_{130}H_{118}O_{16}P_4$ = 2060,24 g/Mol): C 75,47 (75,79); H 5,50 (5,77); P 6,23 (6,01)%. $^{31}$P-NMR ($CD_2Cl_2$): 129,5 (s) ppm. $^{1}$H-NMR ($CD_2Cl_2$): 1,34 (36 H); 1,90 (6 H); 3,13 (4 H); 3,89 (4 H), 7,05...7,87 (84 H) ppm. ESI-TOF HRMS: *m/e* 2082,72637 (M+Na)$^+$.

**Ligand VIII:**

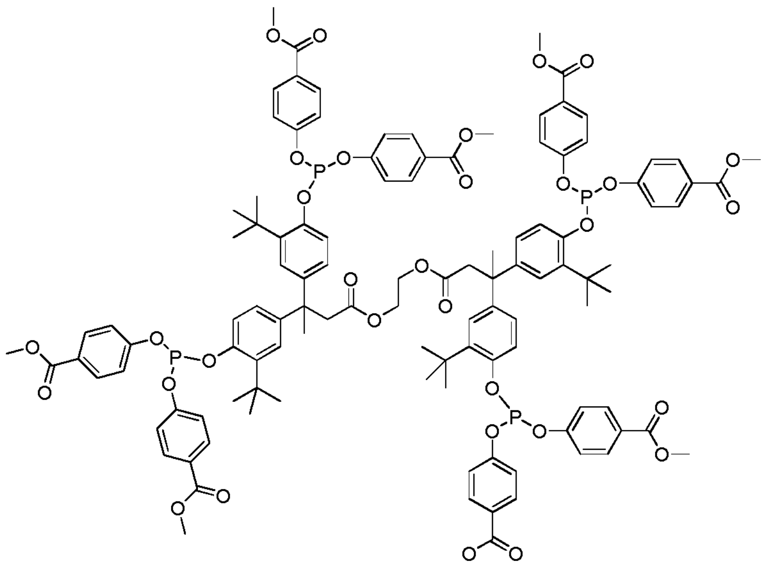

Eine Lösung der Zwischenstufe IV (0,734 g; 0,613 mmol) in Toluol (6 ml) wird bei 0 °C tropfenweise unter Rühren mit einer Lösung von 4-Hydroxybenzoesäuremethylester (0,746 g; 4,901 mmol) in einer Mischung aus Toluol (14 ml) und Triethylamin (6,2 ml) versetzt. Man lässt über Nacht bei Raumtemperatur rühren, filtriert und engt das Filtrat im Vakuum ein. Der erhaltene Rückstand wird zunächst 2 h bei 50°C / 0,1 mbar getrocknet und dann in trockenem, siedendem Acetonitril (10 ml) aufgenommen. Lagerung der Lösung bei -23 °C ergibt 0,847 g (0,399 mmol; 65%) des Tetraphosphites. Analyse (ber. für $C_{114}H_{118}O_{32}P_4$ = 2124,05 g/Mol): C 64,50 (64,46); H 5,50 (5,60); P 5,85 (5,83)%. $^{31}$P-NMR ($CD_2Cl_2$): 128,4 (s) ppm. $^{1}$H-NMR ($CD_2Cl_2$): 1,29 (36 H); 1,88 (6 H); 3,13 (4 H); 3,89 (4 H), 3,91 (24 H); 6,87...8,06 (44 H) ppm. ESI-TOF HRMS: *m/e* 2146,64606 (M+Na)$^+$.

**Ligand IX:**

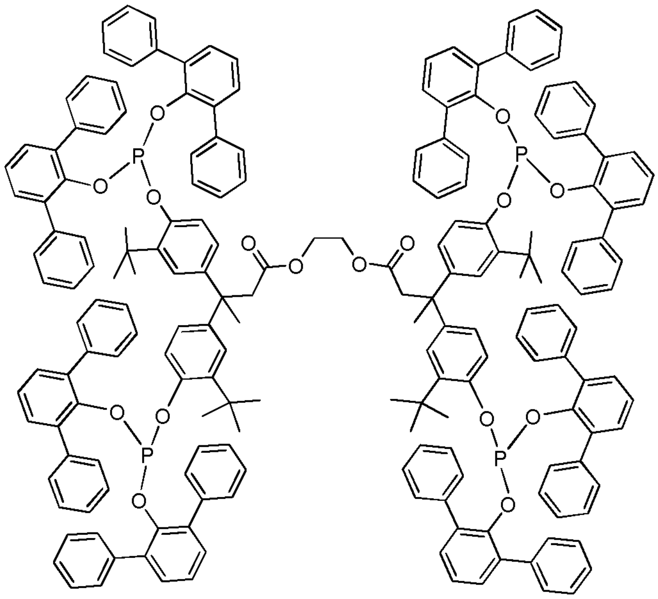

Eine Lösung der Zwischenstufe IV (0,573 g; 0,478 mmol) in Toluol (4 ml) wird bei 0 °C tropfenweise unter Rühren mit einer Lösung von 2,6-Diphenylphenol (0,942 g; 3,826 mmol) in einer Mischung aus Toluol (12 ml) und Triethylamin (5,9 ml) versetzt. Man rührt zunächst über Nacht bei Raumtemperatur, anschließend 4 h bei 70 °C und noch 2 h bei 100 °C Badtemperatur. Man filtriert und engt das Filtrat im Vakuum ein. Der erhaltene Rückstand wird 2 h bei 50°C / 0,1 mbar getrocknet. Die säulenchromatografische Reinigung (Hexan/Dichlormethan (1:1), $R_f$= 0,16) ergibt 0,392 g (0,136 mmol; 28%) des Tetraphosphites. Analyse (ber. für $C_{194}H_{166}O_{16}P_4$ = 2877,32 g/Mol): C 80,81 (80,98); H 5,92 (5,81); P 4,19 (4,31)%. $^{31}$P-NMR ($CD_2Cl_2$): 142,4 (s) ppm. $^1$H-NMR ($CD_2Cl_2$): 1,05 (36 H); 1,82 (6 H); 3,04 (4 H); 4,04 (4 H); 5,79...7,64 (116 H) ppm. ESI-TOF HRMS: *m/e* 2900,11232 (M+Na)$^+$.

**Ligand X:**

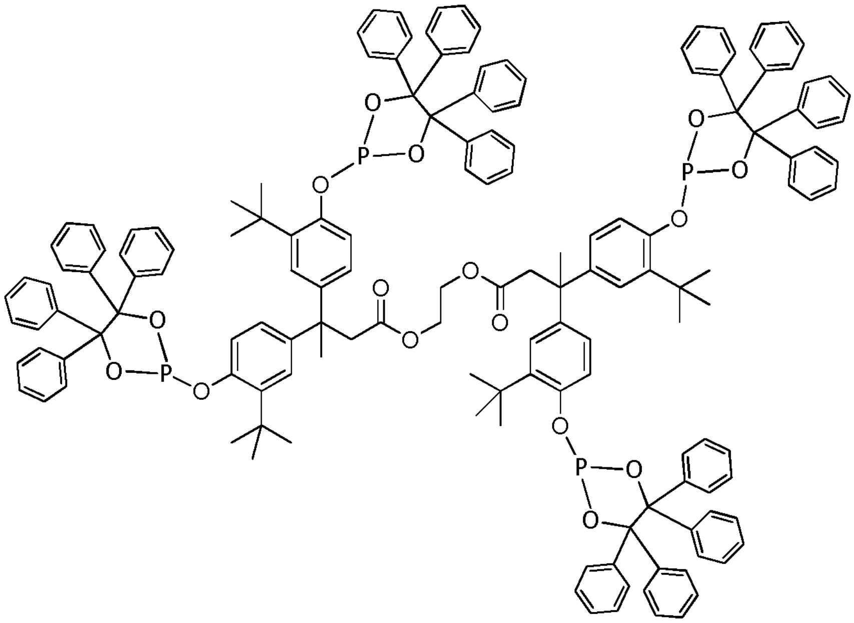

Eine gerührte Lösung von 2-Chlor-4,4,5,5-tetraphenyl-1,3,2-dioxaphospholan (1,074 g; 2,493 mmol) in THF (6 ml) wird bei 0 °C tropfenweise mit einer Lösung von Hostanox 03 (0,451 g; 0,567 mmol) in Triethylamin (0,71 ml) und THF (7 ml) versetzt. Man rührt zunächst über Nacht bei Raumtemperatur und anschließend 10 h bei 70 °C, filtriert und

engt das Filtrat im Vakuum ein. Der erhaltene Rückstand wird 2 h bei 50°C / 0,1 mbar getrocknet und anschließend aus Acetonitril kristallisiert. Ausbeute: 0,66 g (0,278 mmol; 49%) an 92%-igem Produkt (Bilanz: P-NMR). $^{31}$P-NMR ($CD_2Cl_2$): 138,5 (s) ppm. $^1$H-NMR ($CD_2Cl_2$): 1,20 (36 H); 1,90 (6 H); 3,15 (4 H); 3,91 (4 H); 6,92...7,63 (92 H) ppm. ESI-TOF HRMS: *m/e* 2393,91863 (M+Na)$^+$.

**Ligand XI:**

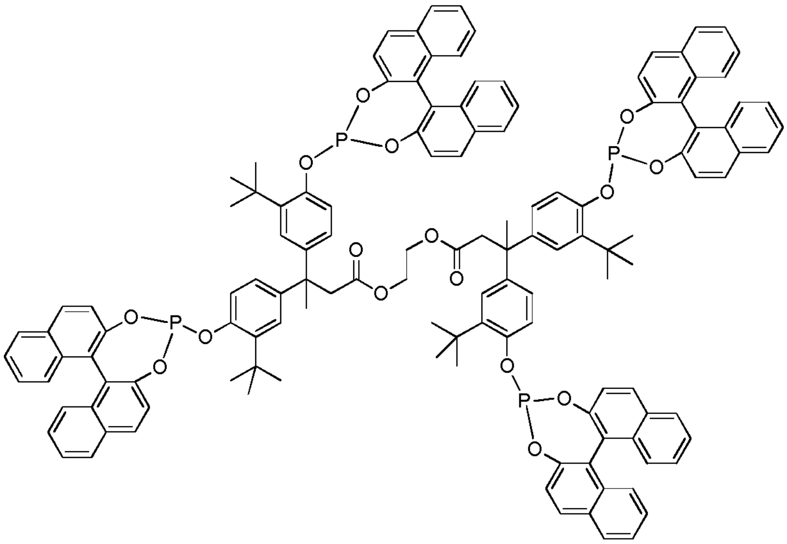

Eine gerührte Lösung von *R*-4-Chlorodinaphtho[2,1-d:1',2'-f][1,3,2]dioxaphosphepin (1,143 g; 3,259 mmol) in THF (8 ml) wird bei 0 °C tropfenweise mit einer Lösung von Hostanox 03 (0,589 g; 0,741 mmol) in Triethylamin (0,93 ml) und THF (9 ml) versetzt. Man rührt über Nacht bei Raumtemperatur, filtriert und engt das Filtrat im Vakuum ein. Der erhaltene Rückstand wird 2 h bei 50°C / 0,1 mbar getrocknet. Die säulenchromatografische Reinigung (Dichlormethan, $R_f$ = 0,71) ergibt 1,128 g (0,550 mmol; 74%) des Tetraphosphites.

Analyse (ber. für $C_{130}H_{110}O_{16}P_4$ = 2052,15 g/Mol):C 75,90 (76,09); H 5,56 (5,40); P 6,14 (6,04)%. $^{31}$P-NMR ($CD_2Cl_2$): 145,6 (s) ppm. $^1$H-NMR ($CD_2Cl_2$): 1,34 (36 H); 1,91 (6 H); 3,16 (4 H); 3,93 (4 H); 7,07...8,08 (60 H) ppm.

**Ligand XII:**

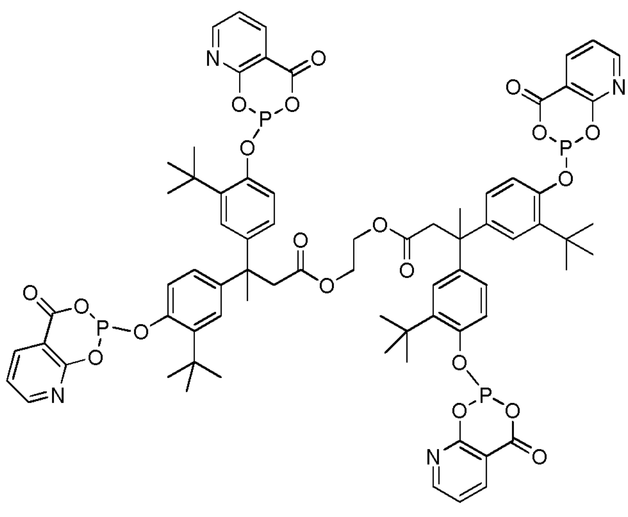

Eine Lösung der Zwischenstufe IV (1,121 g; 0,936 mmol) in Toluol (7 ml) wird bei 0 °C tropfenweise unter Rühren mit einer Lösung von 2-Hydroxynicotinsäure (0,506 g; 3,742 mmol) in einer Mischung aus Toluol (13 ml) und Triethylamin (5,6 ml) versetzt. Man rührt über Nacht bei Raumtemperatur, filtriert und engt das Filtrat im Vakuum ein. Der erhaltene Rückstand wird 2 h bei 50°C / 0,1 mbar getrocknet und wie erhalten verwendet. Ausbeute: 0,67 g (0,458 mmol; 49 %). Analyse (ber. für $C_{74}H_{74}N_4O_{20}P_4$ = 1463,30 g/Mol): C 60,92 (60,74); H 5,02 (5,10); P 8,57 (8,47) %. $^{31}$P-NMR ($CD_2Cl_2$): 115,1 (s) ppm. $^1$H-NMR ($CD_2Cl_2$): 1,18 (36 H); 1,84 (6 H); 3,08 (4 H); 3,85 (4 H); 7,00...8,63 (24 H) ppm.

**Ligand XIII:**

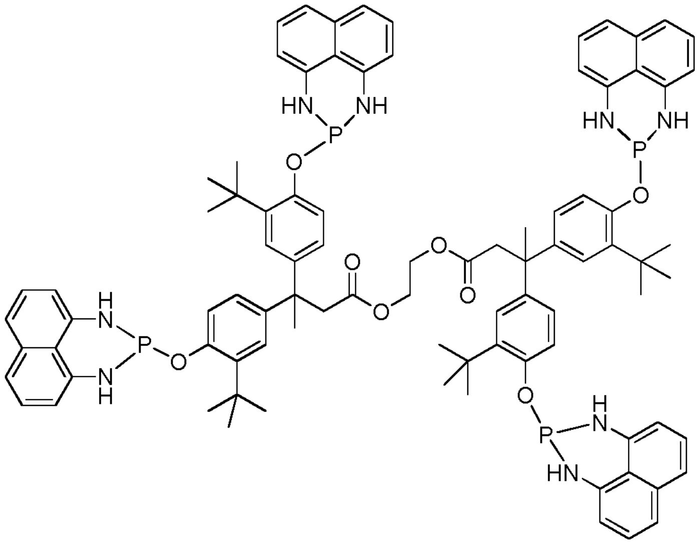

Eine Lösung der Zwischenstufe IV (1,356 g; 1,144 mmol) in Toluol (9 ml) wird bei 0 °C tropfenweise unter Rühren mit einer Mischung von 1,8-Diaminonaphthalin (0,724 g; 4,577 mmol), Toluol (18 ml) und Triethylamin (7,1 ml) versetzt. Man rührt über Nacht bei Raumtemperatur, filtriert und engt das Filtrat im Vakuum ein. Der erhaltene Rückstand wird 2 h bei 50°C / 0,1 mbar getrocknet und wie erhalten verwendet. Ausbeute: 0,500 g (0,325 mmol; 28 %). Analyse (ber. für $C_{90}H_{94}N_8O_8P_4$ = 1539,68 g/Mol): C 70,46 (70,21); H 6,15 (6,15); P 7,95 (8,05) %. $^{31}$P-NMR ($CD_2Cl_2$): 82,2 (s) ppm. $^1$H-NMR ($CD_2Cl_2$): 1,04 (36 H); 1,82 (6 H); 3,08 (4 H); 3,65 (4 H); 5,72 (m, 8 H); 6,51...7,25 (36 H) ppm. ESI-TOF HRMS: *m*/*e* 1577,57657 (M+Na)$^+$.

**Ligand XIV:**

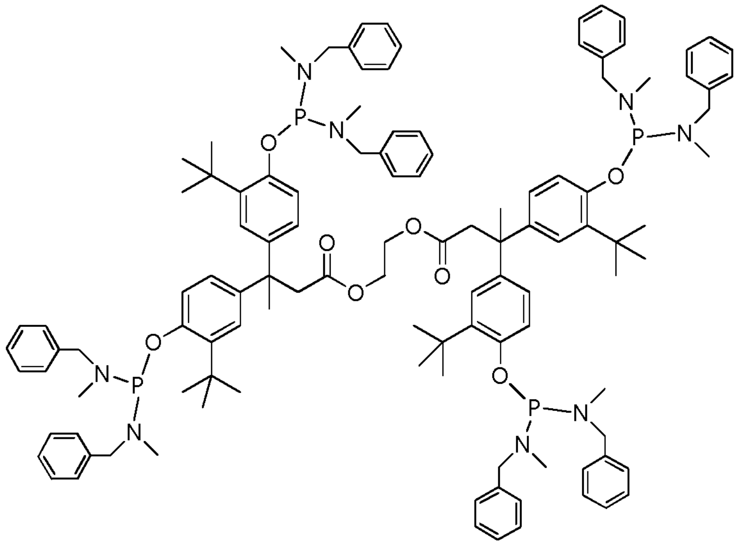

Eine Lösung der Zwischenstufe IV (1,585 g; 1,323 mmol) in Toluol (20 ml) wird bei 0 °C tropfenweise unter Rühren mit einer Lösung von N-Benzylmethylamin (1,282 g; 10,581 mmol) in einer Mischung aus Toluol (17 ml) und Triethylamin (13,4 ml) versetzt. Man rührt über Nacht bei Raumtemperatur, filtriert und engt das Filtrat im Vakuum ein. Der erhaltene Rückstand wird 2 h bei 50°C / 0,1 mbar getrocknet und anschließend mit Diethylether (10 ml) verrührt. Filtration und Einengen des Filtrates im Vakuum erbringen das Produkt als weißen Feststoff. Ausbeute: 1,62 g (0,863 mmol; 65 %). Analyse (Ber. Für $C_{114}H_{142}N_8O_8P_4$ = 1876,29 g/Mol): C72,83 (72,97); H7,65 (7,63); N6,08 (5,97); P 6,76 (6,60) %. $^{31}$P-NMR ($CD_2Cl_2$): 128,9 (s) ppm.$^1$H-NMR ($CD_2Cl_2$): 1,47 (36 H); 1,97 (6 H); 2,68 (d, $^3J_{HP}$ = 6,9 Hz; 24 H), 3,21 (4 H); 3,97 (4 H); 4,23 (dd, $^3J_{HP}$ = 8,5 Hz; $^2J_{HH}$ = 14,7 Hz; 8 H); 4,40 (dd, $^3J_{HP}$ = 8,5 Hz; $^2J_{HH}$ = 14,7 Hz; 8 H); 7,05...7,39 (52 H) ppm.

Rhodiumkomplex aus **Ligand VI** und [acacRh$(CO)_2$]:

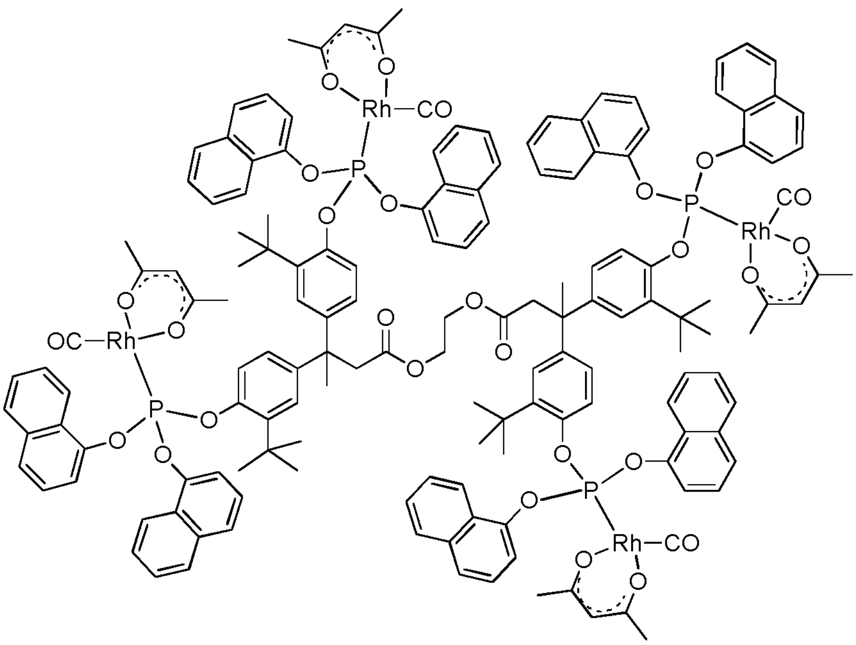

Zu einer gerührten Lösung von [acacRh$(CO)_2$] (acac = acetylacetonat) (0,174 g; 0,672 mmol) in Toluol (2 ml) wird bei Raumtemperatur tropfenweise eine Lösung von Ligand VI (0,315 g; 0,153 mmol) in Toluol (6 ml) gegeben. Man rührt über Nacht und entfernt das Lösungsmittel im Vakuum. Der erhaltene Rückstand wird in siedendem Hexan (10

ml) verrührt. Filtration und Einengen des Filtrates im Vakuum ergibt einen gelben Feststoff. Ausbeute: 0,322 g (0,108 mmol; 70%). Analyse (ber. Für $C_{154}H_{146}O_{28}P_4Rh_4$ = 2980,36 g/Mol): C 62,10 (62,06); H 4,81 (4,94); P 4,12 (4,16); Rh 13,96 (13,81)%. $^{31}$P-NMR ($CD_2Cl_2$): 121,1 (d, $J_{PRH}$ = 291 Hz) ppm.

Durchführung der Katalyseversuche

Die Hydroformylierung wurde in einem mit Druckkonstanthaltung, Gasflussmessung, Begasungsrührer und Druckpipette ausgestattetem 200 ml-Autoklaven der Fa. Premex Reactor AG, Lengau, Schweiz, durchgeführt. Zur Minimierung eines Einflusses von Feuchtigkeit und Sauerstoff wurde das als Solvens benutzte Toluol mit Natrium-Ketyl behandelt und unter Argon destilliert. Die als Substrat eingesetzten Substrate 1-Octen (Aldrich), *cis*/*trans*-2-Penten (Aldrich) und n-Octene (Evonik Industries, Octenisomerengemisch aus 1-Octen: ~3 %;*cis*+*trans*-2-Octen: ~49 %; *cis*+*trans*-3-Octen: ~29 %; *cis*+*trans*-Octen-4: ~16 %; gerüstisomere Octene: ~3 %) wurden mehrere Stunden über Natrium am Rückfluss erhitzt und unter Argon destilliert.

Für die Versuche wurden im Autoklaven unter Argonatmosphäre Lösungen der Katalysatorvorstufe und des Liganden gemischt. Als Katalysatorvorstufe kam [(acac)Rh(COD)] (Umicore, acac = Acetylacetonat-Anion; COD = 1,5-Cyclooctadien) zum Einsatz. Für Versuche mit einer Konzentration von 100 ppm Rhodium wurden 10 ml einer 4,31 millimolaren Lösung, für Versuche mit variierter Rhodiumkonzentration das gleiche Volumen entsprechend konzentrierter Lösungen in den Autoklaven gegeben. Anschließend wurde die entsprechende Masse des Hostanoxliganden, in der Regel 1 Äquivalent pro Rhodium, in 10 ml Toluol gelöst und zugemischt. Durch Zugabe von weiterem Toluol wurde das Anfangsvolumen der Katalysatorlösung auf 41,0 ml eingestellt. In eine druckfeste Pipette wurde als Olefin eingefüllt: n-Octene (10,70 g; 95,35 mmol), 1-Octen (10,54 g; 93,92 mmol) oder 2-Penten (9,75 g; 139,00 mmol). Der Autoklav wurde bei einem Gesamtgasdruck (Synthesegas: Linde; $H_2$ (99,999%): $CO_2$ (99,997%) = 1:1) von a) 42 bar für einen Enddruck von 50 bar; b) 12 bar für den Enddruck von 20 bar unter Rühren (1500 U/min) aufgeheizt. Nach Erreichen der Reaktionstemperatur von 120 °C wurde der Synthesegasdruck auf a) 48,5 bar für einen Enddruck von 50 bar, b) 19,5 bar für einen Enddruck von 20 bar erhöht und das Olefin(-gemisch) mit einem in der Druckpipette eingestellten Überdruck von ca. 3 bar in den Autoklaven gepresst. Die Reaktion wurde bei konstantem Druck von jeweils 50 bar bzw. 20 bar (Nachdruckregler der Fa. Bronkhorst, NL) über 4 h geführt. Der Autoklav wurde nach Ablauf der Reaktionszeit auf Zimmertemperatur abgekühlt, unter Rühren entspannt und mit Argon gespült. Jeweils 1 ml der Reaktionsmischungen wurden unmittelbar nach Abschalten des Rührers entnommen, mit 5 ml Pentan verdünnt und gaschromatographisch analysiert: HP 5890 Series II plus, PONA, 50 m x 0,2 mm x 0,5 µm.

Ergebnisse der Katalyseversuche

2-Penten

| Ligand | L/Rh | Ausb. (%) | n-Sel. (%) |
|---|---|---|---|
| **I** | 0,5 | 99 | 52,3 |
| **II** | 1 | 98 | 42,8 |
| **III** | 1 | 99 | 39,0 |
| **III** | 0,5 | 99 | 39,8 |
| **V** | 1 | 99 | 40,7 |
| **VI** | 1 | 99 | 47,1 |
| **VII** | 1 | 99 | 46,0 |
| **VII** | 0,2 | 97 | 50,1 |
| Reaktionsbedingungen: T = 120 °C, p = 20 bar $CO/H_2$, t = 4 h, [Rh] = 100 ppm-m, Lösungsmittel: Toluol | | | |

n-Octen

| Ligand | P (bar) | [Rh] ppm-m | L/Rh | Ausb. (%) | n-Sel. (%) |
|---|---|---|---|---|---|
| **I** | 50 | 100 | 0,2 | 94 | 29,4 |

(fortgesetzt)

| Ligand | P (bar) | [Rh] ppm-m | L/Rh | Ausb. (%) | n-Sel. (%) |
|---|---|---|---|---|---|
| **I** | 50 | 100 | 0,5 | 93 | 27,7 |
| **II** | 20 | 100 | 1 | 83 | 33,1 |
| **II** | 50 | 100 | 1 | 93 | 26,7 |
| **III** | 50 | 100 | 0,2 | 98 | 26,3 |
| **III** | 50 | 100 | 0,5 | 96 | 24,3 |
| **III** | 20 | 100 | 1 | 94 | 29,8 |
| **III** | 50 | 100 | 1 | 96 | 21,7 |
| **III** | 50 | 80 | 1 | 98 | 22,7 |
| **III** | 50 | 60 | 1 | 97 | 23,1 |
| **III** | 50 | 40 | 1 | 96 | 24,3 |
| **III** | 50 | 10 | 1 | 95 | 27,4 |
| **III** | 50 | 100 | 2 | 99 | 19,9 |
| **III** | 50 | 100 | 5 | 98 | 16,5 |
| **V** | 50 | 100 | 1 | 98 | 19,3 |
| **VI** | 50 | 100 | 1 | 98 | 19,8 |
| **VII** | 50 | 100 | 1 | 95 | 16,9 |
| **VII** | 20 | 100 | 0,2 | 97 | 16,9 |
| Reaktionsbedingungen: T = 120 °C, t = 4 h, Lösungsmittel: Toluol<br>**1-Octen** mit **Ligand III,** 100°C, 50 bar, 40 ppm Rhodium, Ligand: Rh = 1 : 1, Toluol als Lösungsmittel, 4 h Reaktionszeit. Einsatz 1-Octen: 93,92 mmol; Ausbeute Aldehyd: 98,4%. n-Selektivität: 47,2% | | | | | |

Besonders interessant ist die Tatsache, dass der Katalysator mit der neuen Ligandklasse in unerwarteter Weise stabilisiert wird. Nur 0,2 Äquivalente des Liganden, d. h. nur 0,8 P-Atome pro Rhodiumatom, reichen aus, um einen hochaktiven Katalysator zu bilden welcher unter Reaktionsbedingungen nicht zur thermischen Zersetzung neigt. Es werden insbesondere keine Rh(0)-Carbonyle abgeschieden, was ein bekannter Prozess der Inaktivierung technischer Hydroformylierungsprozesse ist. Die mögliche niedrige Dosierung eröffnet den Weg zur Steigerung der Selektivität auf über 50 % beim Einsatz kurzkettiger interner Olefine, wie z.B. 2-Penten. Eine höhere Dosierung des Liganden ist zudem ebenfalls nicht schädlich. Mit 5 Äquivalenten, d. h. mit 20 P-Atomen pro Rhodiumatom, ist ebenfalls eine effektive Katalyse möglich. Die hier beschriebenen Liganden erlauben den Einsatz sehr geringer Rhodiumkonzentrationen. Selbst bei 10 ppm Rh, ist ein nahezu vollständiger und chemoselektiver Umsatz der n-Octene möglich.

Versuchsbeschreibung Autoklaven:

Die Versuche wurden in 100 ml-Autoklaven der Fa. Parr Instrument durchgeführt. Die Autoklaven sind mit einer elektrischen Beheizung ausgerüstet. Die Druckkonstanthaltung erfolgt über Massedurchflußmesser und Druckregler. Während der Versuchszeit kann über eine Spritzenpumpe eine genau definierte Eduktmenge unter Reaktionsbedingungen eingespritzt werden. Über Kapillarleitungen und HPLC-Ventile können während der Versuchszeit Proben gezogen und sowohl über GC- als auch über LC-MS-Analytik untersucht werden.

Beispiel 1

In einem 100 ml-Autoklaven der Fa. Parr Instruments wurde bei 120 °C und 20 bar 6.6 g cis-2-Buten hydroformyliert. Als Precursor wurden 0.0124 g $Rh(acac)(CO)_2$ in 42.39 g getrocknetem Toluol vorgelegt. Als Ligand wurden 0.0585 g Ligand III in der Katalysatoransatzlösung eingesetzt (Ligand:Rh = 0.48 (molar)). Es wurden 0.0108 g Sebacinsäuredi-4-(2,2,6,6-tetramethylpiperidinyl)ester als Stabilisator und 1.8295 g Mesitylen als GC-Standard zugefügt. Das Edukt wurde nach Erreichen der vorgesehenen Reaktionstemperatur zudosiert.

Während der Reaktion wurde der Druck über eine Synthesegasregelung mit Massedurchflussmesser konstant gehalten. Nach 12 Stunden Reaktionszeit wurden 23.0 mol-% Pentanal, 29.7 mol-% 2-Methylbutanal und 3.14 mol-% n-Butan gebildet. Die Regioselektivität zu n-Pentanal beträgt 43.6 %.

Beispiel 2

In einem 100 ml-Autoklaven der Fa. Parr Instruments wurde bei 120 °C und 20 bar 6.5 g cis-2-Buten hydroformyliert. Als Precursor wurden 0.0133 g Rh(acac)$(CO)_2$ in 41.95 g getrocknetem Toluol vorgelegt. Als Ligand wurden 0.1202 g Ligand III (Ligand:Rh = 0.91(molar)) in der Katalysatoransatzlösung eingesetzt. Es wurden 0.0236 g Sebacinsäuredi-4-(2,2,6,6-tetramethylpiperidinyl)ester als Stabilisator und 1.7899 g Mesitylen als GC-Standard zugefügt. Das Edukt wurde nach Erreichen der vorgesehenen Reaktionstemperatur zudosiert.

Während der Reaktion wurde der Druck über eine Synthesegasregelung mit Massedurchflussmesser konstant gehalten. Nach 12 Stunden Reaktionszeit wurden 33.5 mol-% Pentanal, 47.5 mol-% 2-Methylbutanal, 2.89 mol% n-Pentanol und 1.77 mol-% n-Butan gebildet. Die Regioselektivität zu n-Pentanal beträgt 41.4 %.

Beispiel 3

In einem 100 ml-Autoklaven der Fa. Parr Instruments wurde bei 120 °C und 20 bar 6.2 g cis-2-Buten hydroformyliert. Als Precursor wurden 0.0123 g Rh(acac)$(CO)_2$ in 45.00 g getrocknetem Toluol vorgelegt. Als Ligand wurden 0.0420 g Ligand I (Ligand:Rh = 0.53 (molar)) in der Katalysatoransatzlösung eingesetzt. Es wurden 0.0117 g Sebacinsäuredi-4-(2,2,6,6-tetramethylpiperidinyl)ester als Stabilisator und 1.7348 g Mesitylen als GC-Standard zugefügt. Das Edukt wurde nach Erreichen der vorgesehenen Reaktionstemperatur zudosiert.

Während der Reaktion wurde der Druck über eine Synthesegasregelung mit Massedurchflussmesser konstant gehalten. Nach 12 Stunden Reaktionszeit wurden 44.0 mol-% Pentanal, 38.5 mol-% 2-Methylbutanal, 1.24 mol% n-Pentanol und 1.31 mol-% n-Butan gebildet. Die Regioselektivität zu n-Pentanal beträgt 53.3 %.

Beispiel 4

In einem 100 ml-Autoklaven der Fa. Parr Instruments wurde bei 120 °C und 20 bar 6.3 g cis-2-Buten hydroformyliert. Als Precursor wurden 0.0123 g Rh(acac)$(CO)_2$ in 44.19 g getrocknetem Toluol vorgelegt. Als Ligand wurden 0.0784 g Ligand I (Ligand:Rh = 1.0 (molar)) in der Katalysatoransatzlösung eingesetzt. Es wurden 0.0230 g Sebacinsäuredi-4-(2,2,6,6-tetramethylpiperidinyl)ester als Stabilisator und 1.6984 g Mesitylen als GC-Standard zugefügt. Das Edukt wurde nach Erreichen der vorgesehenen Reaktionstemperatur zudosiert.

Während der Reaktion wurde der Druck über eine Synthesegasregelung mit Massedurchflussmesser konstant gehalten. Nach 12 Stunden Reaktionszeit wurden 16.6 mol-% Pentanal, 15.5 mol-% 2-Methylbutanal und 0.44 mol-% n-Butan gebildet. Die Regioselektivität zu n-Pentanal beträgt 51.7 %.

Beispiel 5

In einem 100 ml-Autoklaven der Fa. Parr Instruments wurde bei 120 °C und 50 bar 6.5 g cis-2-Buten hydroformyliert. Als Precursor wurden 0.0119 g Rh(acac)$(CO)_2$ in 42.54 g getrocknetem Toluol vorgelegt. Als Ligand wurden 0.0550 g Ligand III (Ligand:Rh = 0.47 (molar)) in der Katalysatoransatzlösung eingesetzt. Es wurden 0.0121 g Sebacinsäuredi-4-(2,2,6,6-tetramethylpiperidinyl)ester als Stabilisator und 0.4836 g 1,2,4,5-Tetraisopropylbenzol (TIPB) als GC-Standard zugefügt. Das Edukt wurde nach Erreichen der vorgesehenen Reaktionstemperatur zudosiert.

Während der Reaktion wurde der Druck über eine Synthesegasregelung mit Massedurchflussmesser konstant gehalten. Nach 12 Stunden Reaktionszeit wurden 34.4 mol-% Pentanal, 63.1 mol-% 2-Methylbutanal und 0.57 mol-% n-Butan gebildet. Die Regioselektivität zu n-Pentanal beträgt 35.2 %.

Beispiel 6

In einem 100 ml-Autoklaven der Fa. Parr Instruments wurde bei 120 °C und 50 bar 6.3 g cis-2-Buten hydroformyliert. Als Precursor wurden 0.0124 g Rh(acac)$(CO)_2$ in 44.64 g getrocknetem Toluol vorgelegt. Als Ligand wurden 0.1185 g Ligand III (Ligand:Rh = 0.97 (molar)) in der Katalysatoransatzlösung eingesetzt. Es wurden 0.0228 g Sebacinsäuredi-4-(2,2,6,6-tetramethylpiperidinyl)ester als Stabilisator und 0.5083 g 1,2,4,5-Tetraisopropylbenzol (TIPB) als GC-Standard zugefügt. Das Edukt wurde nach Erreichen der vorgesehenen Reaktionstemperatur zudosiert.

Während der Reaktion wurde der Druck über eine Synthesegasregelung mit Massedurchflussmesser konstant gehalten. Nach 12 Stunden Reaktionszeit wurden 29.6 mol-% Pentanal, 59.1 mol-% 2-Methylbutanal, 2,71 mol% n-Pentanol und 0.57 mol-% n-Butan gebildet. Die Regioselektivität zu n-Pentanal beträgt 33.4 %.

Beispiel 7

In einem 100 ml-Autoklaven der Fa. Parr Instruments wurde bei 120 °C und 50 bar 6.3 g cis-2-Buten hydroformyliert. Als Precursor wurden 0.0126 g $Rh(acac)(CO)_2$ in 41.34 g getrocknetem Toluol vorgelegt. Als Ligand wurden 0.0376 g Ligand I (Ligand:Rh = 0.47 (molar)) in der Katalysatoransatzlösung eingesetzt. Es wurden 0.0108 g Sebacinsäuredi-4-(2,2,6,6-tetramethylpiperidinyl)ester als Stabilisator und 0.4977 g 1,2,4,5-Tetraisopropylbenzol (TIPB) als GC-Standard zugefügt. Das Edukt wurde nach Erreichen der vorgesehenen Reaktionstemperatur zudosiert.

Während der Reaktion wurde der Druck über eine Synthesegasregelung mit Massedurchflussmesser konstant gehalten. Nach 12 Stunden Reaktionszeit wurden 35.5 mol-% Pentanal, 56.1 mol-% 2-Methylbutanal, 2,07 mol% n-Pentanol und 0.51 mol-% n-Butan gebildet.

Die Regioselektivität zu n-Pentanal beträgt 38.8 %.

Beispiel 8

In einem 100 ml-Autoklaven der Fa. Parr Instruments wurde bei 120 °C und 50 bar 6.1 g cis-2-Buten hydroformyliert. Als Precursor wurden 0.0120 g $Rh(acac)(CO)_2$ in 43.19 g getrocknetem Toluol vorgelegt. Als Ligand wurden 0.0775 g Ligand I (Ligand:Rh = 1.01 (molar)) in der Katalysatoransatzlösung eingesetzt. Es wurden 0.0233 g Sebacinsäuredi-4-(2,2,6,6-tetramethylpiperidinyl)ester als Stabilisator und 0.4961 g 1,2,4,5-Tetraisopropylbenzol (TIPB) als GC-Standard zugefügt. Das Edukt wurde nach Erreichen der vorgesehenen Reaktionstemperatur zudosiert.

Während der Reaktion wurde der Druck über eine Synthesegasregelung mit Massedurchflussmesser konstant gehalten. Nach 12 Stunden Reaktionszeit wurden 6.59 mol-% Pentanal, 18.2 mol-% 2-Methylbutanal und 0.13 mol-% n-Butan gebildet. Die Regioselektivität zu n-Pentanal beträgt 26.6 %.

Beispiel 9

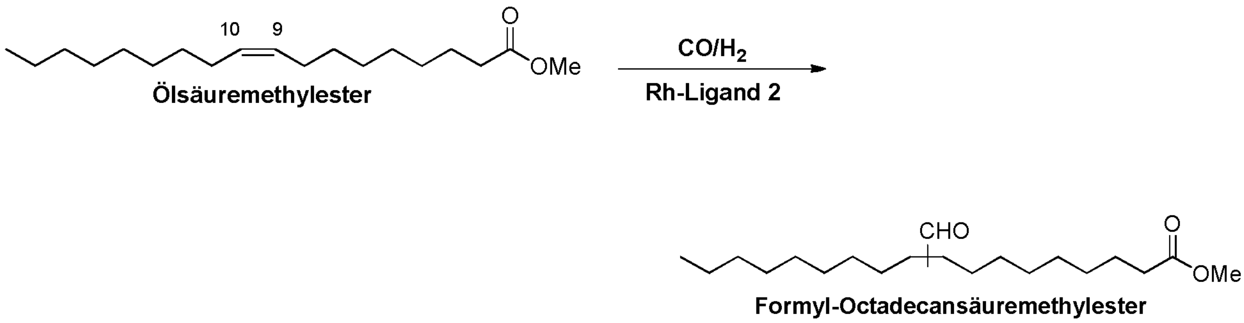


Ölsäuremethylester

Formyl-Octadecansäuremethylester

$[Rh(acac)(CO)_2]$ (1,4 mg, 5,43 μmol) wurde unter Argon in einem Schlenkgefäß eingewogen und in 5 mL Toluol gelöst. 1 mL von dieser Lösung mit Ölsäuremethylester (von Sigma Aldrich) (1,0 mmol, 0,296 g), Ligand II (6,9 μmol für Rh:L=1:6,25 und 27,5 μmol für Rh:L=1:25), Octadecan (0,100 g) und 9 mL Toluol wurden in einen 25-mL Autoklav gefüllt. Der Autoklav wurde dreimal mit Stickstoff (10 bar), einmal mit Synthesegas ($CO:H_2$ = 1:1, 10 bar) sekuriert und dann auf 80 °C geheizt und der Druck auf 20 bar eingestellt. Nach 6 h Reaktionszeit wird der Autoklav gekühlt. Anschließend wurde der Druck bei Raumtemperatur abgelassen und es wurde zweimal mit Stickstoff gespült. Aus dem Autoklaven wurde eine Probe für GC/MS Analyse entnommen. Das Lösungsmittel wurde unter vermindertem druck entfernt und das gelbe Öl wurde per NMR analysiert.

| Rh:L | Rh:Phosphor | Umsatz [%] | Ausbeute [%] (Formyl-Octadecansäuremethylester) |
|---|---|---|---|
| 1:6,25 | 1:25 | 98,0 | 81,9 |
| 1:25 | 1:100 | 98,6 | 85,5 |

Ligand II:

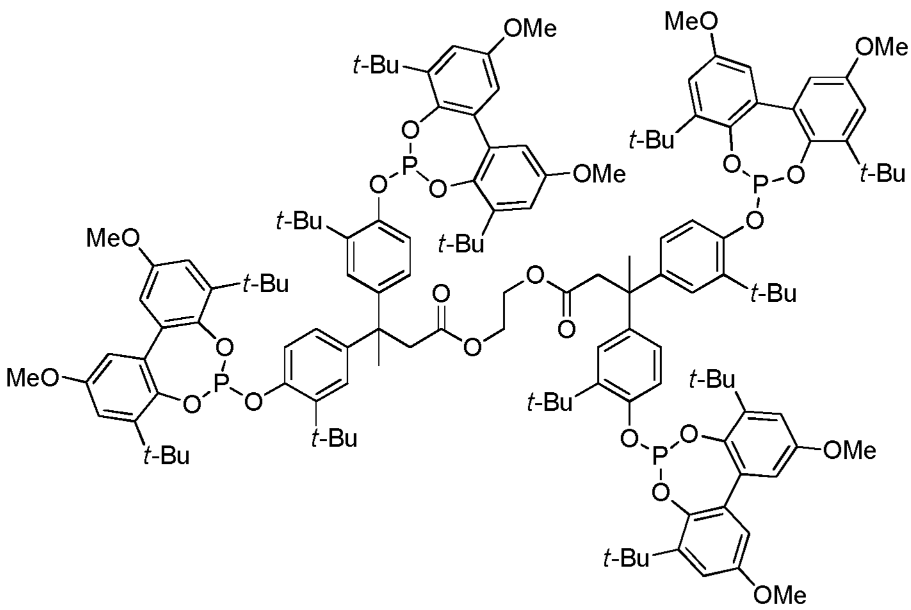

Anhand des Beispiels des Ölsäuremethylesters konnte gezeigt werden, dass mit den erfindungsgemäßen Liganden auch langkettige Olefine hydroformyliert und dass funktionelle Gruppen toleriert werden und das Produkt, der Formyl-Octandecansäuremethylester, in sehr guten Ausbeuten erhalten werden kann.

Vergleichsversuch

Zu Vergleichszwecken wurden Experimente mit dem folgenden nicht-erfindungsgemäßen Vergleichsliganden durchgeführt:

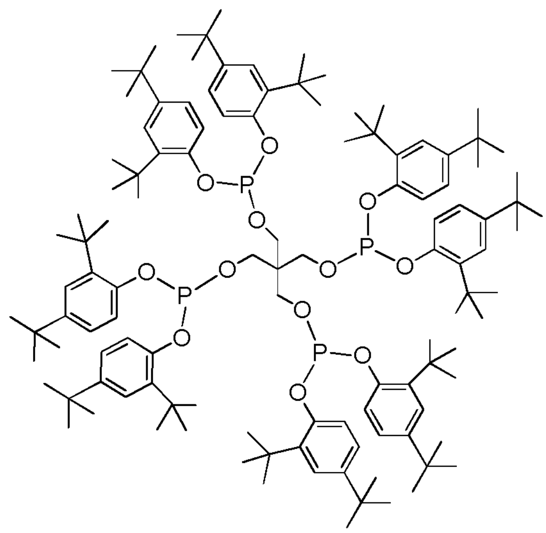

Der Vergleichsligand wurde analog der Vorschrift im Patent EP0518241, Formel 35 hergestellt.

Die Versuchsbedingungen entsprechen denen, welche auch bei den erfindungsgemäßen Liganden angewendet wurden.

2-Penten: 20 bar, 120 °C, 4 h, L/Rh 1:1 ⇒ Ausbeute 17 %
20 bar, 120 °C, 4 h, L/Rh 0,5:1 ⇒ Ausbeute 43 %

n-Octen: 50 bar, 120 °C, 4 h, L/Rh 1:1 ⇒ Ausbeute 6 %
20 bar, 120 °C, 4 h, L/Rh 1:1 ⇒ Ausbeute 10 %

Bei dem eingesetzten Vergleichsliganden handelt es sich ebenfalls um ein Tetraphosphit. Hierbei wurden jedoch unter vergleichbaren Versuchsbedingungen deutlich schlechtere Ausbeuten als mit den erfindungsgemäßen Liganden erzielt werden. So wurden mit dem Vergleichsligand lediglich maximal 43 % Ausbeute an dem Zielprodukt, dem Aldehyd, erhalten, wohingegen bei Verwendung der erfindungsgemäßen Liganden in allen Fällen Ausbeuten von > 90 % erzielt werden konnten. Die technische Aufgabe wurde somit unter Verwendung der erfindungsgemäßen Liganden erfüllt.

Ligandenrückhalteversuche

Die Grundtechniken auf dem Gebiet der Membranfiltration beschreiben Melin / Rautenbach: Membranverfahren: Grundlagen der Modul- und Anlagenauslegung. Springer, Berlin Heidelberg 2004.

Nanofiltration ist ein druckgetriebenes Membrantrennverfahren. Die Trenngrenze (engl.: molecular weight cut-off, MWCO; vgl. Y.H. See Toh, X.X. Loh, A. Bismarck, A.G. Livingston, In search of a standard method for the characterisation of organic solvent nanofiltration membranes, J. Membr. Sci, 291(2007)120-125]) liegt im Bereich von 150 g/mol bis 2000 g/mol. Über diesen Wert lässt sich die Nanofiltarion von anderen Membrantrennverfahren wie Mikrofiltration und Ultrafiltration abgrenzen. Die Trenngrenze ist definiert als die Molmasse eines bevorzugt inerten Indikatorsystems (z. B. Polystyrolstandards oder Alkanstandards bei Toh, Loh, Bismarck und Livingston), bei der eine Membran einen Rückhalt von 90% aufweist. Die genaue Trenngrenze einer Nanofiltrationsmembran ist von der eingesetzten Membran und dem jeweiligen Lösemittel sowie durch die Prozessbedingungen wie Druck und Temperatur bestimmt. Bei der Nanofiltration werden dichte oder poröse Membranen eingesetzt. Nanofiltrationsmembranen zeichnen sich durch einen geringen Rückhalt für niedermolekulare organische Stoffe aus.

Der Rückhalt R einer Membran ist über die örtlichen Konzentrationen einer Komponente i des nicht permeierenden Stroms (Retentat) sowie des durch die Membran permeierenden Stroms (Permeat) bestimmt. Sind Retentat und Permeat entlang der Membran ideal durchmischt, so entsprechen die örtlichen Retentat- und Permeatkonzentrationen der jeweiligen Konzentration des in der Summe anfallenden Retentats bzw. Permeats. Der Rückhalt R einer Membran für eine im zugeführten Stoffstrom enthaltene Komponente i ist wie folgt definiert:

$$R = 1 - c_{Pei}/c_{Rei}$$

Dabei ist $c_{Pei}$ die Konzentration der Komponente i im Permeat (Pe) und $c_{Rei}$ die Konzentration der Komponente i im Retentat (Re). Im Grenzfall eines vollständigen Rückhalts der Komponente i durch die Membran ist $c_{Pei}= 0$ und R = 1. Im Fall einer bevorzugten Permeation der Komponente i ist $c_{Pei} > c_{Rei}$, und $R < 0$.

Für die Trennaufgabe geeignet erscheinen Membranen, die eine trennaktive Schicht aus einem Material, ausgewählt aus Celluloseacetat, Cellulosetriacetat, Cellulosenitrat, regenerierter Cellulose, Polyimiden, Polyamiden, Polyetheretherketonen, sulfonierten Polyetheretherketonen, aromatischen Polyamiden, Polyamidimiden, Polybenzimidazolen, Polybenzimidazolone, Polyacrylnitril, Polyarylethersulfone, Polyester, Polycarbonaten, Polytetrafluorethylen, Polyvinylidenfluorid, Polypropylen, Polydimethylsiloxan, Silicone, Polyphosphazene, Polyphenylsulfide, Polybenzimidazole, 6.6 Nylon, Polysulfone, Polyaniline, Polyurethane, Acrylonitril/Glycidylmethacrylat (PANGMA), Polytrimethylsilylpropyne, Polymethylpentyne, Polyvinyltrimethylsilan, alpha-Aluminiumoxide, Titaniumoxide, gamma-Aluminiumoxide, Polyphenylenoxid, Siliciumoxide, Zirconiumoxide, mit Silanen hydrophobisierte keramische Membranen, wie sie in DE10308111 beschrieben sind, Polymere mit intrinsischer Mikroporösität (PIM) wie PIM-1 und anderen, wie sie z. B. in EP0781166 und in "Membranes" von I. Cabasso, Encyclopedia of Polymer Sience and Technlogy, John Wiley and Sons, New York, 1987, beschrieben werden, aufweisen. Die oben genannten Stoffe können insbesondere in der trennaktiven Schicht ggf. durch Zugabe von Hilfsstoffen vernetzt vorliegen oder als so genannte Mixed Matrix Membranes mit Füllstoffen wie z. B. Carbon Nano Tubes, Metal Organic Frameworks oder Hollow Spheres sowie Partikel von anorganischen Oxiden oder anorganische Fasern, wie z. B. Keramik- oder Glasfasern versehen sein.

Bevorzugt werden Membranen eingesetzt, die als trennaktive Schicht eine Polymerschicht aus Polydimethylsiloxan, Polyimid, Polyamidimid, Acrylonitril/Glycidylmethacrylat (PANGMA), Polyamid oder Polyetheretherketon aufweisen, die aus Polymeren mit intrinsischer Mikroporosität (PIM) wie PIM-1 aufgebaut sind, oder wobei die trennaktive Schicht über eine hydrophobierte keramische Membran aufgebaut ist. Ganz besonders bevorzugt werden Membranen aus Silikonen oder Polyamidimid eingesetzt. Solche Membranen sind kommerziell erhältlich.

Beispiel Ligandenrückhalteversuch mit Ligand III:

Die Filtrationsversuche wurden in einer gerührten Dead-End Trennzelle der Firma Evonik Membrane Extraction Technology Limited vom Typ METCELL® durchgeführt. Verwendet wurde eine organophile Nanofiltrationsmembran

der Firma GMT Membrantechnik GmbH vom Typ ONF-2. Die Membranfläche beträgt 54 $cm^2$. Die aktive Membranfläche ist dem Feed zugewandt verbaut. Das Permeat fließt durch die Sintermetalplatte der Trennzelle über den Permeatauslass ab.

Der Ligand III wurde mit einer Konzentration von 4,0 mg/ml in 250 ml getrocknetem Toluol in einem sekurierten Schlenk gelöst und unter inerten Bedingungen in die Trennzelle überführt. Die Filtration wurde bei 20 °C und einem Stickstoffdruck von 10 bar durchgeführt.

Vom erzeugten Permeat wurden zunächst 25,0 g als Vorlauf verworfen. In der anschießenden Permeatprobe von 18,3 g ist kein Ligand nachweisbar. Die Nachweisgrenze liegt bei 0,1 ml/g. Weitere 86,8 g Permeat wurden verworfen und danach wurde eine weitere Permeatprobe von 12,7 g vermessen. Auch in dieser Probe liegt die Ligandkonzentration unterhalb der Nachweisgrenze. Das bedeutet, dass der Ligand vollständig zurückgehalten wird. Der Katalysator wurde auf eine Konzentration von 12,91 mg/ml aufkonzentriert. Diese Konzentration entspricht dem volumetrischen Konzentrierungsfaktor.

In WO 2011/107441 A1 wird die Abtrennung der katalytisch wirksamen Zusammensetzung mittels organischer Nanofiltration und Rückführung in die Hydroformylierungsreaktion beschrieben. Die katalytisch aktive Zusammensetzung enthält dabei POSS-modifizierte Liganden. Diese aktive Zusammensetzung, also Ligand und Metallkomplex wurde gut, jedoch nicht vollständig zurückgehalten.

In dem oben beschriebenen erfindungsgemäßen Beispiel konnte gezeigt werden, dass im Fall der Ethylen-bis[3,3-bis(3-tert-butyl-4-hydroxyphenyl)butyrat]-modifizierten Liganden ein vollständiger Rückhalt bereits ohne Bindung an den Metallkomplex, also in freier Form und nicht als katalytisch aktive Zusammensetzung, erfolgte. Dieser Ligandentyp stellt also folglich eine Verbesserung gegenüber dem Stand der Technik dar und erfüllt somit die technische Aufgabe.

## Patentansprüche

1. Ligand, welcher die folgende Struktur **III** aufweist:

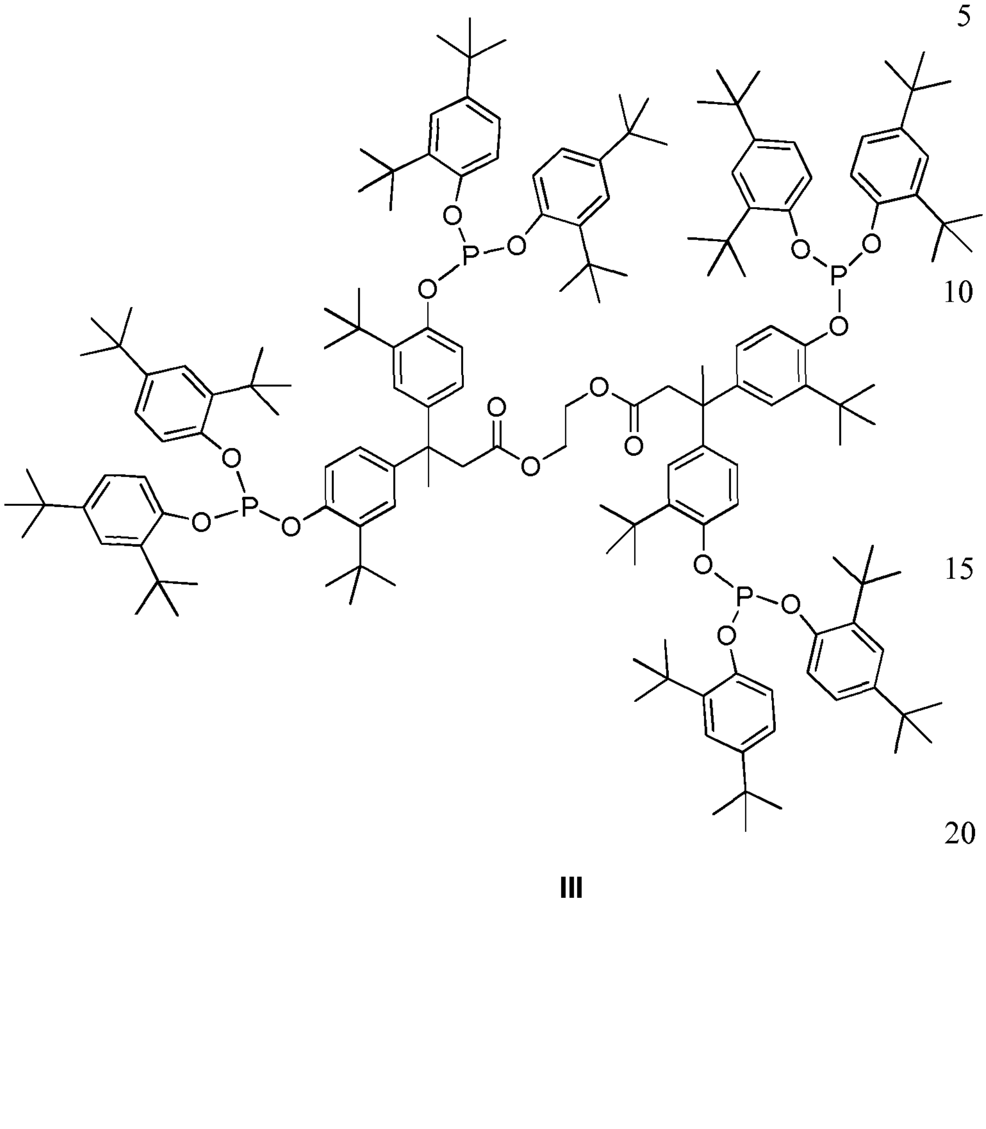

**III**

## Claims

**1.** Ligand having the following structure **III:**

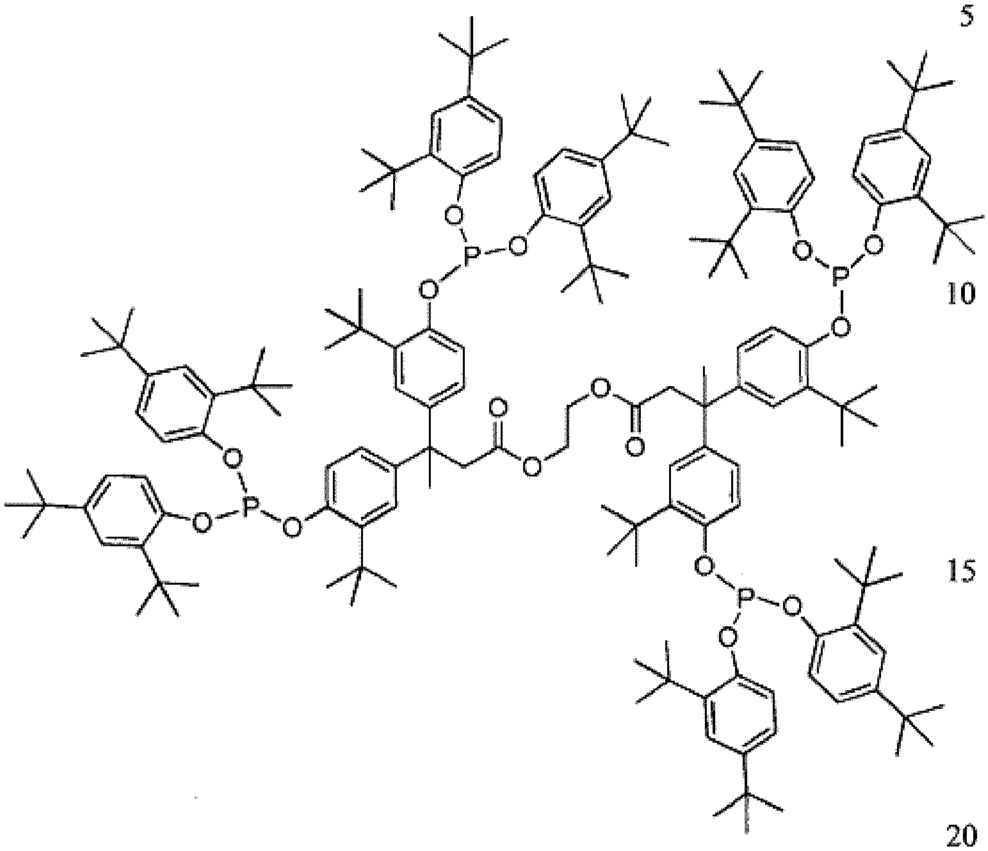

**III**

## Revendications

**1.** Ligand, présentant la structure III suivante :

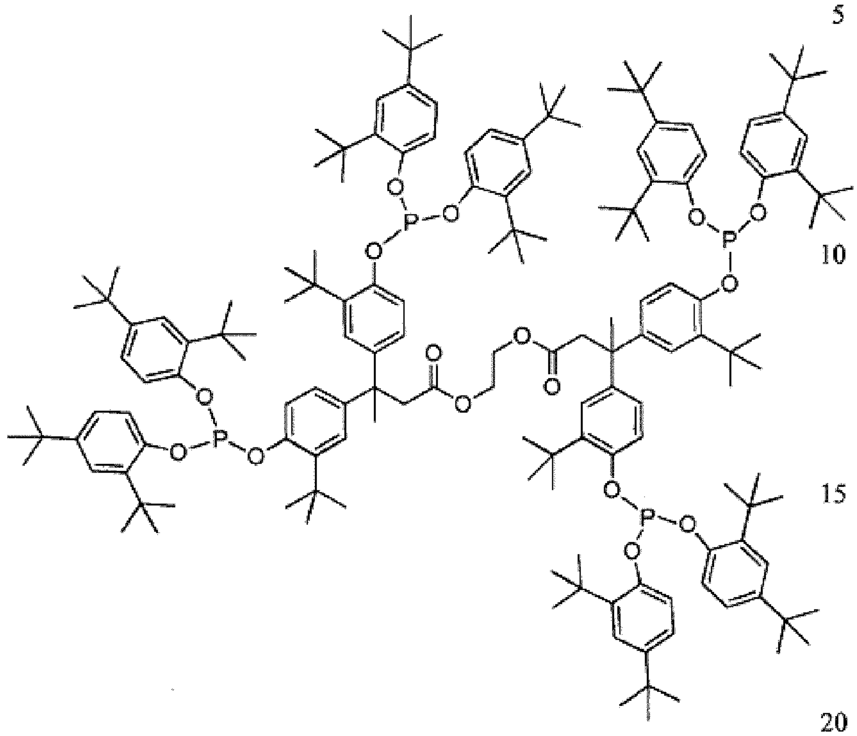

III

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Patentdokumente


- US 4694109 A
- US 4879416 A
- WO 9530680 A
- DE 10140083 A1
- EP 0518241 A
- DE 10308111
- EP 0781166 A
- WO 2011107441 A1


### In der Beschreibung aufgeführte Nicht-Patentliteratur


- **B. CORNILS ; W. A. HERRMANN.** Applied Homogeneous Catalysis with Organometallic Compounds. VCH, 1996, vol. 1 & 2
- **R. FRANKE ; D. SELENT ; A. BÖRNER.** Applied Hydroformylation. *Chem. Rev.*
- *CHEMISTRY - A EUROPEAN JOURNAL,* 2002, vol. 8 (15), 3343
- **PRISKE, M. et al.** Reaction integrated separation of homogeneous catalysts in the hydroformylation of higher olefins by means of organophilic nanofiltration. *Journal of Membrane Science,* 15. September 2010, vol. 360 (1-2), 77-83
- **R. FRANKE ; D. SELENT ; A. BÖRNER.** Applied Hydroformylation. *Chem. Rev.,* 2012
- **P. W. N. M. VAN LEEUWEN.** Rhodium Catalyzed Hydroformylation. Kluwer, 2000
- **K.D. WIESE ; D. OBST.** *Top. Organomet. Chem.,* 2006, vol. 18, 1-13
- **W. L. F. ARMAREGO ; CHRISTINA CHAI.** Purification of Laboratory Chemicals. Butterworth Heinemann (Elsevier), 2009
- **ROBIN K. HARRIS ; EDWIN D. BECKER ; SONIA M. CABRAL DE MENEZES ; ROBIN GOODFELLOW ; PIERRE GRANGER.** *Pure Appl. Chem.,* 2001, vol. 73, 1795-1818
- **ROBIN K. HARRIS ; EDWIN D. BECKER ; SONIA M. CABRAL DE MENEZES ; PIERRE GRANGER ; ROY E. HOFFMAN ; KURT W. ZILM.** *Pure Appl. Chem.,* 2008, vol. 80, 59-84
- **MELIN ; RAUTENBACH.** Grundlagen der Modul- und Anlagenauslegung. Springer, 2004
- **Y.H. SEE TOH ; X.X. LOH ; A. BISMARCK ; A.G. LIVINGSTON.** search of a standard method for the characterisation of organic solvent nanofiltration membranes. *J. Membr. Sci,* 2007, vol. 291, 120-125
- Membranes. **I. CABASSO.** Encyclopedia of Polymer Sience and Technlogy. John Wiley and Sons, 1987